# EUROPEAN PATENT APPLICATION

(11) **EP 3 131 055 A1**
(43) Date of publication of application: **15.02.2017**
(21) Application number: 16178224.8
(22) Date of filing: 06.07.2016
(51) Int. Cl.: G06Q 50/24

(54) **MEDICAL ASSISTANCE DEVICE, OPERATION METHOD AND OPERATION PROGRAM THEREOF, AND MEDICAL ASSISTANCE SYSTEM**

(30) Priority: 13.08.2015 JP 2015159787
(71) Applicant: FUJIFILM Corporation, Tokyo 106-8620 (JP)
(72) Inventor: OKABE, Yuuki, Tokyo, Tokyo 107-0052 (JP); KAMODA, Rena, Tokyo, Tokyo 107-0052 (JP)
(74) Representative: Klunker . Schmitt-Nilson . Hirsch

(57) **Abstract**

A work list includes information display cells, in which work information regarding work performed on a patient by medical staff is displayed, and blank cells, in which no work information is displayed. The display mode of the work list is switched to a first display mode in which the blank cells are displayed without being deleted, a second display mode in which the blank cells are deleted so that the information display cells are aggregatively displayed by being packed together in the row direction, and a third display mode in which the blank cells are deleted so that the information display cells are aggregatively displayed by being packed together in the column direction.

## Description

### BACKGROUND OF THE INVENTION

### 1. Field of the Invention

The present invention relates to a medical assistance device, an operation method and operation program thereof, and a medical assistance system.

### 2. Description of the Related Art

In the medical field, a medical assistance device is known that generates a display screen of a work list, in which information regarding a plurality of work pieces (hereinafter, referred to as work information), such as medical examinations performed on a patient, is displayed, for the smooth progress of the treatment for a patient. The medical assistance device provides the generated display screen for the viewing of medical staff, such as a doctor who examines a patient, a nurse who nurses a patient, or a technician who performs a medical examination. The medical staff can check the work information of the work that the medical staff themselves are in charge of through the work list.

For example, JP2006-338521A discloses a work list (denoted as a patient list in JP2006-338521A) configured to include a patient array axis on which patient identification information such as patient names of a plurality of patients is arranged, an item array axis on which items of a plurality of work pieces (hereinafter, referred to as work items) are arranged, and a plurality of cells that are intersections between a plurality of pieces of patient identification information and a plurality of work items. In JP2006-338521A, in a cell corresponding to a work item of work scheduled to be performed or work that has already been performed, among a plurality of cells, the work information of the work is displayed. On the other hand, needless to say, no work information is displayed in a cell corresponding to a work item of work, which is not scheduled to be performed. In addition, in JP2006-338521 A, an icon showing the progress of work is displayed as the work information.

### SUMMARY OF THE INVENTION

In the work list described in JP2006-338521A, the cells in which work information is displayed as described above (hereinafter, referred to as information display cells) and cells in which no work information is displayed (hereinafter, referred to as blank cells) are scattered. In such a display mode in which the information display cells and the blank cells are scattered, the number of information display cells that can be displayed at one time is reduced by the amount of blank cells since the size of the display screen is limited. For this reason, the degree of at-a-glance listing of the work list is lowered.

For example, in a case where there are ten pieces of patient identification information that can fit in the display screen at one time and there are five people for whom certain work is not scheduled to be performed, five cells that are half of ten cells corresponding to the work items of the work are blank cells. Accordingly, even though there is display space for ten cells, only the five cells that are half of the ten cells can be displayed as information display cells corresponding to the work items of the work. Also in a case where there are ten work items that can fit in the display screen at one time and there are five work pieces, which are not scheduled to be performed for a certain patient, by exchanging the patient identification information with work items, the degree of at-a-glance listing of the work list is similarly lowered. That is, blank cells are obstacles to making it possible to check a larger amount of work information at one time on the display screen having a limited size.

In particular, in the case of expressing work information displayed in a cell with characters in a plurality of rows so that the work information can be checked in detail, the size of each cell is increased. Accordingly, the influence of the lowering in the degree of at-a-glance listing of the work list due to blank cells becomes noticeable.

Nevertheless, the display mode in which information display cells and blank cells are scattered is suitable for a case in which taking a look at the general overview of the work information is desired since it is possible to grasp to which patient identification information and work item each cell corresponds depending on the position of the cell.

The preferred display mode of the work list differs depending on the purpose of checking of the work information by the medical staff, for example, a purpose of checking of the work information focusing on each patient such as a medical examination of a patient, or a purpose of checking of the work information focusing on taking a look at the general overview of the implementation status of work, such as how much work remains, at the start or end of work of medical facilities, or a purpose of checking the work information focusing on checking the work that each member of professional medical staff is in charge of. Therefore, a method has been required that can display the work list in a display mode that each medical staff member desires according to various purposes.

It is an object of the invention to provide a medical assistance device, an operation method and operation program thereof, and a medical assistance system capable of displaying the work list in a display mode that a medical staff member desires according to various purposes.

In order to solve the aforementioned problem, a medical assistance device of the invention comprises: a screen generation unit that generates a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed; a receiving unit that receives designation of either the row direction or the column direction input through the display screen; and a screen display control unit that has an aggregating display function of aggregatively displaying the information display cells spatially by deleting at least one of the blank cells such that the information display cells are packed together in the row direction or the column direction received by the receiving unit and a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.

It is preferable that the receiving unit receives designation for sequentially switching the display mode among the first display mode, the second display mode, and the third display mode by the same input operation through the display screen.

It is preferable that the receiving unit receives at least one of designation of one of a plurality of rows of the work list, designation of one of a plurality of columns of the work list, and designation of one of the plurality of cells.

Preferably, the screen display control unit performs the aggregating display by deleting the blank cells in the one row received by the receiving unit such that the information display cells in the one row are packed together in the row direction and switches the display mode to the second display mode by deleting an entire column regardless of presence or absence of the information display cell for a column in which the blank cell is present, and/or the screen display control unit performs the aggregating display by deleting the blank cells in the one column received by the receiving unit such that the information display cells in the one column are packed together in the column direction and switches the display mode to the third display mode by deleting an entire row regardless of presence or absence of the information display cell for a row in which the blank cell is present.

Alternatively, preferably, the screen display control unit performs the aggregating display by deleting the blank cells in the one row received by the receiving unit such that the information display cells in the one row are packed together in the row direction and switches the display mode to the second display mode by not performing the aggregating display for rows other than the one row received by the receiving unit, and/or the screen display control unit performs the aggregating display by deleting the blank cells in the one column received by the receiving unit such that the information display cells in the one column are packed together in the column direction and switches the display mode to the third display mode by not performing the aggregating display for columns other than the one column received by the receiving unit. In this case, it is preferable that the screen display control unit highlights the one row aggregatively displayed in the second display mode, and/or highlights the one column aggregatively displayed in the third display mode.

Preferably, in a case where the one cell received by the receiving unit is the information display cell, the screen display control unit switches the display mode to the second display mode or the third display mode by packing the information display cells in the row direction or the column direction received by the receiving unit with the one cell being located at a center of the information display cells.

Preferably, the screen display control unit switches the display mode to the second display mode by performing the aggregating display for all of a plurality of rows of the work list, and/or switches the display mode to the third display mode by performing the aggregating display for all of a plurality of columns of the work list.

Preferably, the display mode switching function includes a function of switching the display mode either to the second display mode, in which the blank cells in the one row received by the receiving unit are deleted such that the information display cells in the one row are aggregatively displayed by being packed together in the row direction and no aggregating display is performed for rows other than the one row received by the receiving unit, or to the second display mode in which the aggregating display is performed for all of a plurality of rows of the work list and/or a function of switching the display mode either to the third display mode, in which the blank cells in the one column received by the receiving unit are deleted such that the information display cells in the one column are aggregatively displayed by being packed together in the column direction and no aggregating display is performed for columns other than the one column received by the receiving unit, or to the third display mode in which the aggregating display is performed for all of a plurality of columns of the work list.

It is preferable that, in the second display mode or the third display mode, the screen display control unit displays the work item or the patient identification information corresponding to a case of an original position in the information display cell shifted from the original position in the first display mode due to the aggregating display.

It is preferable that, in the second display mode or the third display mode, in a case where the aggregatively displayed information display cells do not fit in the display screen at one time, the screen display control unit displays the information display cells so as to be folded.

It is preferable that the work information includes a progress of the work. Preferably, as the progress, there are "incomplete", which indicates that the work is scheduled to be performed but has not yet been completed, and "complete", which indicates that the work has been completed. Preferably, the information display cells include an incompletion cell in which the progress is the "incomplete" and a completion cell in which the progress is the "complete".

It is preferable that, in a case where the one cell received by the receiving unit is the blank cell or the incompletion cell, the screen display control unit packs the information display cells in the row direction or the column direction in which the patient array axis is disposed.

It is preferable that, in a case where the one cell received by the receiving unit is the completion cell, the screen display control unit displays details of the work.

Alternatively, it is preferable that, in a case where the one cell received by the receiving unit is the completion cell, the screen display control unit packs the information display cells in the row direction or the column direction in which the item array axis is disposed.

It is preferable that the screen display control unit deletes the blank cell and the completion cell and aggregatively displays only the incompletion cell.

It is preferable that, in a case of packing the information display cells in the row direction or the column direction in which the patient array axis is disposed, the screen display control unit sorts the information display cells in execution order of the work.

An operation method of a medical assistance device of the invention comprises: a screen generation step of generating a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed; a receiving step of receiving designation of either the row direction or the column direction input through the display screen; and a screen display control step of executing an aggregating display function of aggregatively displaying the information display cells spatially by deleting at least one of the blank cells such that the information display cells are packed together in the row direction or the column direction received in the receiving step and executing a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.

An operation program of a medical assistance device of the invention causes a computer to execute: a screen generation function of generating a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed; a receiving function of receiving designation of either the row direction or the column direction input through the display screen; and a screen display control function of executing an aggregating display function of aggregatively displaying the information display cells spatially by deleting at least one of the blank cells such that the information display cells are packed together in the row direction or the column direction received through the receiving function and executing a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.

A medical assistance system of the invention comprises a medical assistance device and a client terminal connected to the medical assistance device through a network. The medical assistance system comprises: a screen generation unit that generates a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed; a receiving unit that receives designation of either the row direction or the column direction input through the display screen; and a screen display control unit that has an aggregating display function of aggregatively displaying the information display cells spatially by deleting at least one of the blank cells such that the information display cells are packed together in the row direction or the column direction received by the receiving unit and a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.

According to the invention, the display mode of the work list in which work information regarding a plurality of work pieces performed on a patient is displayed is switched to the first display mode in which the blank cells are displayed without being deleted, the second display mode in which the blank cells are deleted so that the information display cells are aggregatively displayed by being packed together in the row direction, and the third display mode in which the blank cells are deleted so that the information display cells are aggregatively displayed by being packed together in the column direction. Therefore, it is possible to provide a medical assistance device, an operation method and operation program thereof, and a medical assistance system capable of displaying the work list in a display mode that a medical staff member desires according to various purposes.

### BRIEF DESCRIPTION OF THE DRAWINGS

Fig. 1 is a diagram showing a medical assistance system.
Fig. 2 is a diagram showing the content of electronic medical records stored in a medical record DB.
Fig. 3 is a diagram showing the content of an examination image stored in an image DB.
Fig. 4 is a diagram showing the content of a medical report stored in a report DB.
Fig. 5 is a block diagram showing a computer that forms a client terminal and a medical assistance server.
Fig. 6 is a block diagram showing each functional unit of a CPU of a client terminal.
Fig. 7 is a block diagram showing each functional unit of a CPU of a medical assistance server.
Fig. 8 is a diagram showing the content of a work information table stored in a work information DB.
Fig. 9 is a diagram showing a display screen.
Fig. 10 is an enlarged view of a work list.
Fig. 11 is a diagram showing a work list of the first display mode and a work list of the second display mode.
Fig. 12 is a diagram showing a specific example in which a work item corresponding to the case of the original position is displayed in an information display cell shifted from the original position in the first display mode due to aggregating display.
Fig. 13 is a diagram showing how information display cells are packed together in a row direction.
Fig. 14 is a diagram showing a work list of the first display mode and a work list of the third display mode.
Fig. 15 is a diagram showing a specific example in which patient identification information corresponding to the case of the original position is displayed in an information display cell shifted from the original position in the first display mode due to aggregating display.
Fig. 16 is a diagram showing how information display cells are packed together in a column direction.
Fig. 17 is a diagram showing the designation state of a display mode switching button and the display mode of a work list corresponding thereto.
Fig. 18 is a flowchart showing the procedure of the CPU of the medical assistance server.
Fig. 19 is a diagram showing a work list in which work items of an item column are not displayed.
Fig. 20 is a diagram showing a work list in which patient information of the patient column is made inconspicuous by gray-out processing.
Fig. 21 is a diagram showing a work list of the first display mode and a work list of the second display mode in a second embodiment.
Fig. 22 is a diagram showing how information display cells are packed together in the row direction in the second embodiment.
Fig. 23 is a diagram showing a work list of the first display mode and a work list of the third display mode in the second embodiment.
Fig. 24 is a diagram showing how information display cells are packed together in the column direction in the second embodiment.
Fig. 25 is a diagram showing a work list of the first display mode and a work list of the second display mode in a third embodiment.
Fig. 26 is a diagram showing how information display cells are packed together in the row direction in the third embodiment.
Fig. 27 is a diagram showing a work list of the first display mode and a work list of the third display mode in the third embodiment.
Fig. 28 is a diagram showing how information display cells are packed together in the column direction in the third embodiment.
Fig. 29 is a diagram showing a work list of the first display mode and a work list of the second display mode in a fourth embodiment.
Fig. 30 is a diagram showing how information display cells are packed together in the row direction in the fourth embodiment.
Fig. 31 is a diagram showing a work list of the first display mode and a work list of the third display mode in the fourth embodiment.
Fig. 32 is a diagram showing how information display cells are packed together in the column direction in the fourth embodiment.
Fig. 33 is a diagram showing a work list of the first display mode and a work list of the second display mode in a case where the first and third embodiments are implemented in combination.
Fig. 34 is a diagram showing a work list of the first display mode and a work list of the third display mode in a case where the first and third embodiments are implemented in combination.
Fig. 35 is a diagram showing a sixth embodiment in which information display cells that do not fit in a display portion at one time are folded and displayed.
Fig. 36 is a diagram showing a seventh embodiment in which information display cells are packed together in a direction, in which the patient array axis is disposed, in a case where a blank cell or an incompletion cell is designated and the details of work is displayed in a case where a completion cell is designated.
Fig. 37 is a diagram showing an eighth embodiment in which information display cells are packed together in a direction, in which the patient array axis is disposed, in a case where a blank cell or an incompletion cell is designated and information display cells are packed together in a direction, in which the item array axis is disposed, in a case where a completion cell is designated.
Fig. 38 is a diagram showing a ninth embodiment in which blank cells and completion cells are deleted and only incompletion cells are aggregatively displayed.
Fig. 39 is a diagram showing a tenth embodiment in which information display cells are sorted in work execution order.

### DESCRIPTION OF THE PREFERRED EMBODIMENTS

### [First embodiment]

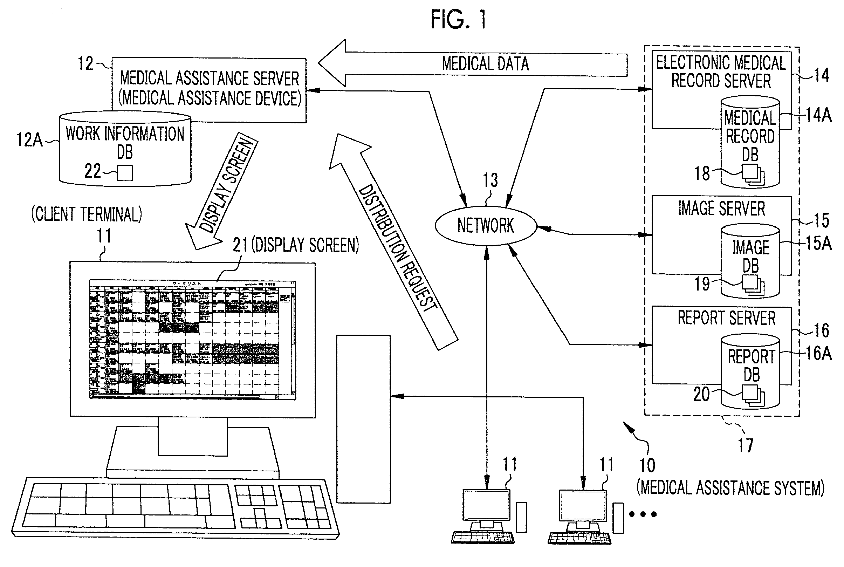

In Fig. 1, a medical assistance system 10 is constructed in a medical facility, and comprises a client terminal 11, a medical assistance server 12 corresponding to a medical assistance device, and the like. The client terminal 11 and the medical assistance server 12 are communicably connected to each other through a network 13, such as a local area network (LAN) provided in the medical facility.

An electronic medical record server 14, an image server 15, and a report server 16 (hereinafter, referred to collectively as a server group 17) are connected to the network 13. The electronic medical record server 14 has a medical record database (hereinafter, abbreviated as a database (DB)) 14A, and the electronic medical record 18 is searchably recorded in the medical record DB 14A. The image server 15 has an image DB 15A, and the examination image 19 obtained by various image examinations is searchably recorded in the image DB 15A. The report server 16 has a report DB 16A, and a medical report 20 in which findings, which are the result of interpretation of the examination image 19 by a radiologist, are summarized, is searchably recorded in the report DB 16A.

Each of the client terminal 11, the medical assistance server 12, and the server group 17 is configured by installing a control program, such as an operating system, or various application programs on a computer as a base, such as a personal computer, a server computer, or a workstation.

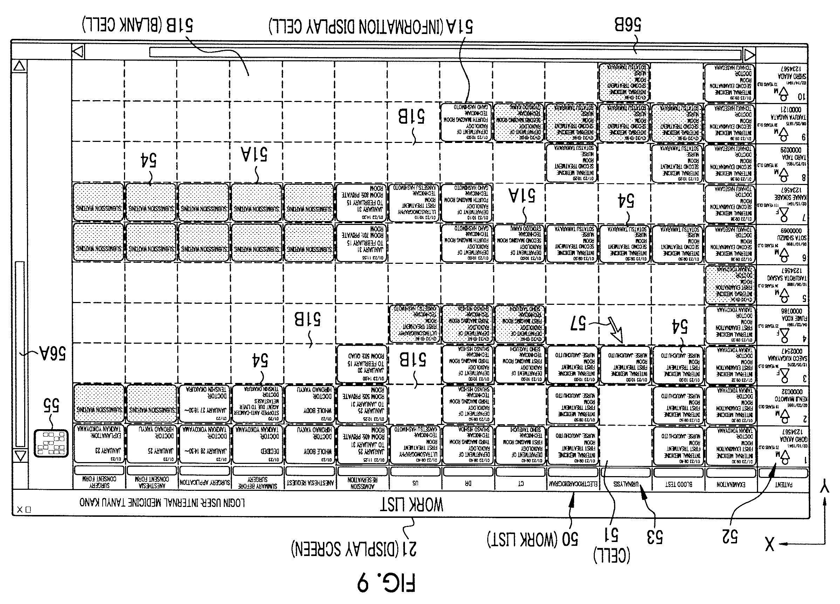

The medical assistance server 12 has a function of outputting a display screen 21 of a work list 50 (refer to Fig. 9 or the like) in which work information regarding a plurality of work pieces, such as medical examinations performed on a patient, is displayed. The work information includes a medical staff member in charge of the work, the progress of work, and the like. The server group 17 has a function of managing the electronic medical record 18, the examination image 19, and the medical report 20.

The client terminal 11 is operated by a doctor who performs medical treatment for a patient, a nurse who nurses a patient, and a laboratory technician or the like who performs various medical examinations (hereinafter, referred to collectively as medical staff). A plurality of client terminals 11 are installed for each department, such as internal medicine, surgery, examination department, and rehabilitation department, or for respective members of the medical staff. The client terminal 11 is used when performing medical treatment for a patient using various functions of the medical assistance server 12 and the server group 17. Specifically, the client terminal 11 is used when viewing the electronic medical record 18, the examination image 19, the medical report 20, or the display screen 21, or when inputting various kinds of information in the electronic medical record 18, or when generating the medical report 20. The client terminal 11 may be a stationary type terminal that is placed in each department, or may be a portable terminal carried by each medical staff member.

The medical assistance server 12 receives a distribution request from the client terminal 11. In addition, the medical assistance server 12 acquires medical data, which is acquired in the medical procedure for a patient who visits a medical facility, from the server group 17. The medical assistance server 12 stores work information corresponding to the acquired medical data in a work information table 22 of a work information DB 12A. The medical assistance server 12 generates the display screen 21 based on the work information of the work information table 22. The medical assistance server 12 transmits the generated display screen 21 to the client terminal 11 that is a source of the distribution request.

The medical assistance server 12 distributes the display screen 21 that can be viewed on the web browser. The medical assistance server 12 issues an authentication key to the client terminal 11 to allow access to the medical assistance server 12. The display screen 21 distributed from the medical assistance server 12 is displayed on the client terminal 11.

The medical assistance server 12 distributes the display screen 21, for example, in the form of image data for web distribution that is created by a markup language, such as Extensible Markup Language (XML). The client terminal 11 reproduces and displays the display screen 21 on the web browser based on the image data. Instead of the XML, other data description languages, such as JAVASCRIPT (registered trademark) Object Notation (JSON), may be used.

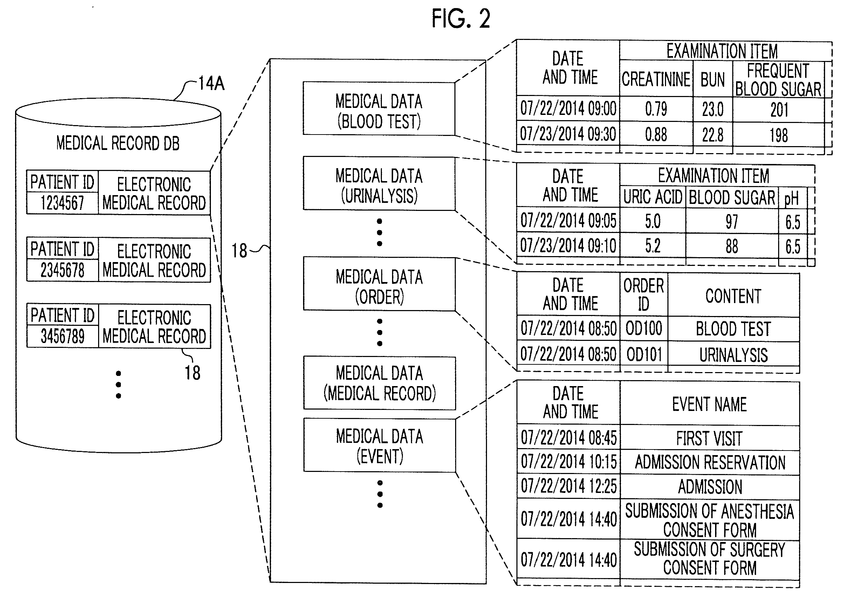

In Fig. 2, the electronic medical record 18 of the medical record DB 14A is managed in units of a patient by being associated with a patient identification data (ID), such as "1234567". The electronic medical record server 14 can find the electronic medical record 18 from the medical record DB 14A by using the patient ID as a search key.

The electronic medical record 18 includes a plurality of types of medical data. The types of medical data include various subject examinations performed on a patient, such as a blood test and urinalysis, orders in which instructions of subject examinations or image examinations, reporting, surgery, medication, and the like are described, medical examination records such as a disease name or the content of a medical examination including the main complaint of the patient that is input to the electronic medical record 18 by the doctor, an event that occurs in the course of medical treatment for the patient, such as a first visit, admission and discharge, anesthesia consent form submission, and surgery consent form submission. Medical data is organized for each of the types and is recorded in time series. The anesthesia consent form and the surgery consent form are documents to explain the need or risk of performing anesthesia and surgery to patients or their families and ask the patients or their families for signatures or stamping.

Records of various kinds of medical data for one case include date and time, such as subject examination date and time, order issuance date and time, medical examination date and time, and event occurrence date and time, and the specific content, such as examination values of a plurality of examination items of subject examinations, an order ID, the content of order, and an event name. For example, in a case where the type of medical data is a blood test, the examination items are creatinine, blood urea nitrogen (BUN), frequent blood sugar, and the like. In a case where the type of medical data is urinalysis, the examination items are uric acid, blood sugar, pH (potential hydrogen; hydrogen ion concentration index), and the like. The order ID is a number or symbol for identifying each order.

In addition to the patient ID, patient information, such as patient's name, sex, age, date of birth, and taste (smoking or drinking), is recorded in the electronic medical record 18. In addition to those described above, the medical data includes vital signs such as the heart rate, pulse, blood pressure, and body temperature of the patient, prescription including drugs administered to the patient and a dose, history, notes such as informative matters between medical staff members or memorandums, and the like are described.

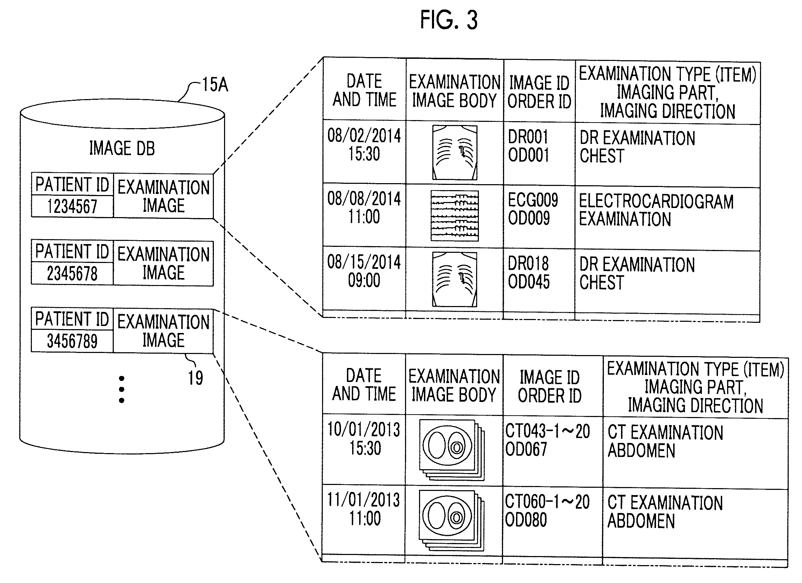

In Fig. 3, the examination image 19 of the image DB 15A is managed in units of a patient by being associated with a patient ID, similarly to the electronic medical record 18. Similarly to the electronic medical record server 14, the image server 15 can find the examination image 19 from the image DB 15A by using the patient ID as a search key.

The examination image 19 is an image obtained by various image examinations. The various image examinations include, for example, an electrocardiogram examination, a computed tomography (CT) examination, a digital radiography (DR) examination, an ultrasonography (ultrasonic; US) examination, a magnetic resonance imaging (nuclear magnetic resonance imaging; MRI) examination, and an endoscopic examination. The examination image 19 of the CT examination, the DR examination, or the MRI examination is generated in the data file format of digital imaging and communications in medicine (DICOM) specification, for example.

The file of the examination image 19 for one case includes the main body of the examination image 19 and various kinds of accessory information including image examination date and time, an image ID and an order ID, types of image examinations such as "DR examination" and "CT examination", an imaging part such as "chest" or "abdomen", and an imaging direction. The types of image examinations are treated as work items. The image server 15 transmits the examination image 19 and such accessory information to the medical assistance server 12 as medical data.

The image ID is a number or symbol for identifying each examination image 19. In the case of an image examination in which a plurality of examination images 19 are captured at one time, such as a CT examination, a common order ID is assigned to each examination image 19 in order to indicate that a plurality of examination images 19 have been obtained by one image examination, and the plurality of examination images 19 are collectively managed as one examination image 19.

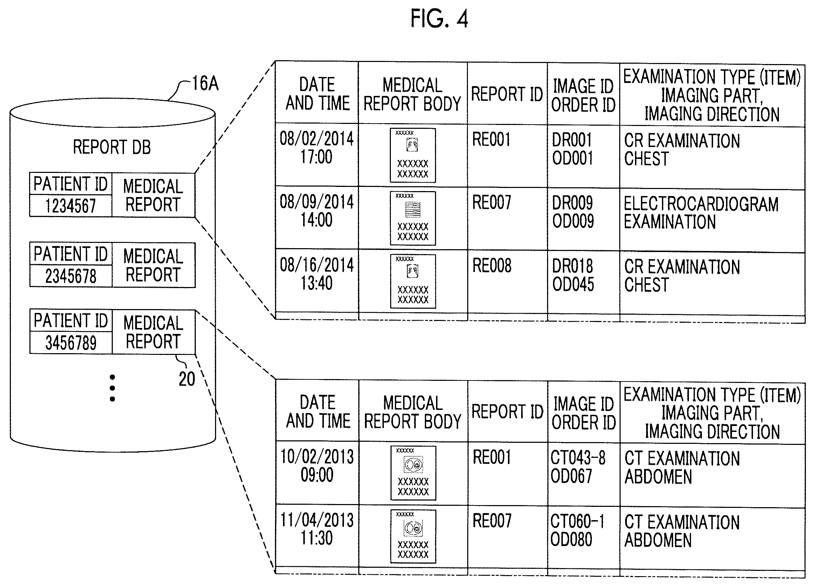

In Fig. 4, the medical report 20 of the report DB 16A is managed in units of a patient by being associated with a patient ID, similarly to the electronic medical record 18 and the examination image 19. Similarly to the servers 14 and 15, the report server 16 can find the medical report 20 from the report DB 16A by using the patient ID as a search key.

The file of the medical report 20 for one case includes the main body of the medical report 20 and various kinds of accessory information including the creation date of the medical report 20, a report ID, an image ID and an order ID of the examination image 19 attached to the medical report 20, types of image examinations, and an imaging part and an imaging direction. The report server 16 transmits the medical report 20 and such accessory information to the medical assistance server 12 as medical data.

In the electronic medical record 18, pieces of information, such as a doctor in charge of a medical examination, a room for medical examination, and an affiliation department or a nurse in charge of a subject examination, a room where a subject examination is performed, and an affiliation department, are stored so as to be associated with each other. In the examination image 19, pieces of information, such as a technician in charge of an image examination, a room for image examination, and an affiliation department, are stored so as to be associated with each other. Based on such information, it can be seen where which member of the medical staff performed what kind of work for which patient.

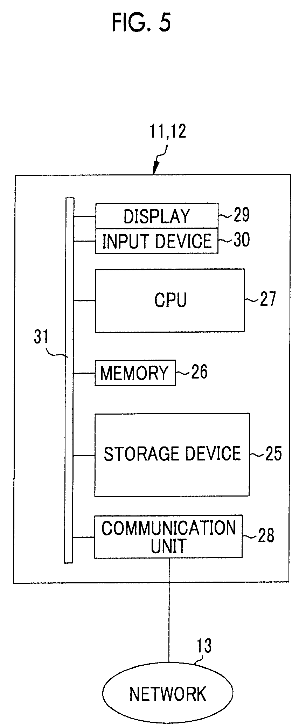

In Fig. 5, the basic configurations of computers that form the client terminal 11 and the medical assistance server 12 are the same, and each computer comprises a storage device 25, a memory 26, a central processing unit (CPU) 27, a communication unit 28, a display 29, and an input device 30. These are connected to each other through a data bus 31.

The storage device 25 is a hard disk drive, which is built into a computer that forms the client terminal 11 or the like or which is connected to the computer through a cable or a network, or a disk array formed by connecting a plurality of hard disk drives. A control program such as an operating system, various application programs, and display data of various screens associated with these programs are stored in the storage device 25.

The memory 26 is a work memory required when the CPU 27 executes processing. The CPU 27 performs overall control of each unit of the computer by loading a program stored in the storage device 25 to the memory 26 and executing the processing according to the program.

The communication unit 28 is a network interface to perform transmission control of various kinds of information through the network 13. The display 29 displays various screens corresponding to the operation of the input device 30, such as a mouse or a keyboard. The screen has an operation function based on the graphical user interface (GUI). Each computer that forms the client terminal 11 or the like receives an input of an operation instruction from the input device 30 through the screen.

In the following explanation, for the sake of distinction, a suffix "A" is attached to the reference numeral of each unit of the computer that forms the client terminal 11, and a suffix "B" is attached to the reference numeral of each unit of the computer that forms the medical assistance server 12.

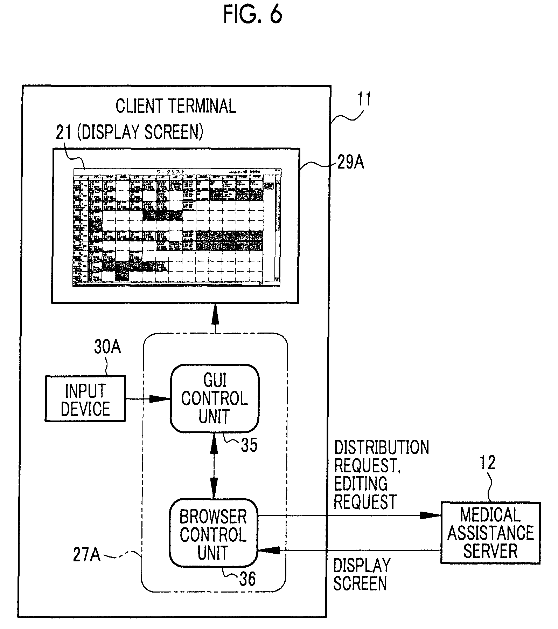

In Fig. 6, when a web browser is started, the CPU 27A of the client terminal 11 cooperates with the memory 26 or the like to function as a GUI control unit 35 and a browser control unit 36.

The GUI control unit 35 displays various screens on the display 29A, and receives an operation instruction that is input from the input device 30A through various screens. As the operation instruction, there are an instruction to distribute the display screen 21 to the medical assistance server 12 and an instruction to edit the display screen 21. The GUI control unit 35 outputs the received operation instruction to the browser control unit 36.

The browser control unit 36 controls the operation of the web browser. The browser control unit 36 issues a distribution request according to the distribution instruction from the GUI control unit 35 and an editing request according to the editing instruction to the medical assistance server 12. The distribution instruction is given, for example, through a distribution instruction screen (not shown) displayed on the display 29A after authentication of the medical assistance server 12.

The browser control unit 36 receives image data of the display screen 21 from the medical assistance server 12. The browser control unit 36 reproduces the display screen 21 to be displayed on the web browser based on the image data, and outputs the display screen 21 to the GUI control unit 35. The GUI control unit 35 displays the display screen 21 on the display 29A.

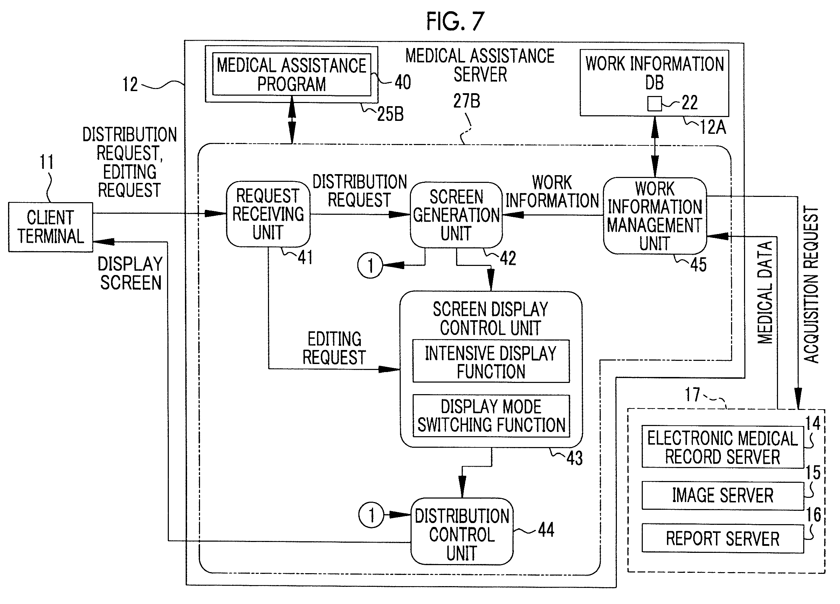

In Fig. 7, a medical assistance program 40 is stored in the storage device 25B of the medical assistance server 12. The medical assistance program 40 is an application program for operating a computer, which forms the medical assistance server 12, as a medical assistance device, and corresponds to the operation program of the medical assistance device.

When the medical assistance program 40 is started, the CPU 27B of the medical assistance server 12 cooperates with the memory 26 or the like to function as a request receiving unit 41, a screen generation unit 42, a screen display control unit 43, a distribution control unit 44, and a work information management unit 45.

The request receiving unit 41 corresponds to a receiving unit. The request receiving unit 41 receives a distribution request and an editing request from the client terminal 11. The request receiving unit 41 outputs the distribution request to the screen generation unit 42, and outputs the editing request to the screen display control unit 43.

The screen generation unit 42 receives work information from the work information management unit 45 in response to the distribution request, and generates the display screen 21 based on the received work information. The screen generation unit 42 outputs the generated display screen 21 to the screen display control unit 43 and the distribution control unit 44.

The screen display control unit 43 edits the display screen 21 from the screen generation unit 42 in response to the editing request. The screen display control unit 43 has an aggregating display function and a display mode switching function as functions for editing the display screen 21. The screen display control unit 43 outputs the edited display screen 21 to the distribution control unit 44.

The distribution control unit 44 outputs the display screen 21 generated by the screen generation unit 42 or the display screen 21 edited by the screen display control unit 43 to the client terminal 11 that is a source of the distribution request or the editing request.

The work information management unit 45 manages the work information table 22 of the work information DB 12A. The work information management unit 45 issues a medical data acquisition request to the server group 17 periodically, for example, every hour. The work information management unit 45 acquires the medical data transmitted from the server group 17 in response to the acquisition request, creates work information based on the acquired medical data, and stores the created work information in the work information table 22. An interval for issuing an acquisition request may be changed according to the type of medical data. For example, a request for the acquisition of medical data relevant to a medical examination may be issued every hour, and a request for the acquisition of other pieces of medical data may be issued every day.

The work information management unit 45 reads work information from the work information table 22, and provides the read work information to the screen generation unit 42 in order to generate the display screen 21.

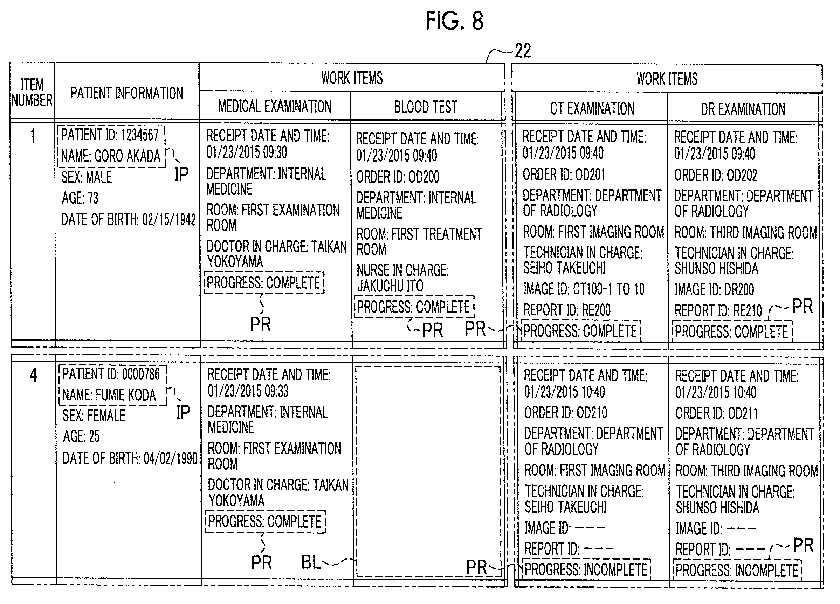

In Fig. 8, in the work information table 22, work information is recorded for each patient. The work information table 22 is divided into an item number, patient information, and a work item. For the item number, for example, the number of an examination reservation order or the number of a reception order is assigned. The above-described patient ID, name, sex, age, and date of birth that are recorded in the electronic medical record 18 are posted in the patient information. Among the pieces of information, a patient ID and a name shown by a dotted frame and a reference numeral IP corresponds to the patient identification information.

Work items are a medical examination, a blood test, a CT examination, a DR examination, and the like. In addition to these, work items of other medical examinations, such as urinalysis, an electrocardiogram examination, and a US examination, and event-related work items, such as admission reservation, an anesthesia request, surgery application, an anesthesia consent form, and a surgery consent form, are included. In each work item, date and time (acceptance date and time) when the work is accepted, a medical department, a room, and a medical staff member in charge of the work are recorded as work information. In the case of a medical examination, an order ID is further recorded. Such pieces of information are posted from the electronic medical record 18 or the examination image 19. In the work item of an image examination, such as a CT examination or a DR examination, in addition to the above-described information including the medical examination, an image ID of the examination image 19 and a report ID of the medical report 20 are posted.

In addition, in the work item, as shown by a dotted frame and a reference numeral PR, the progress of work is recorded as work information. As types of the progress of work, there are "incomplete", which indicates that the work is scheduled to be performed but has not yet been completed as shown in the CT examination of number 4 or the like, and "complete", which indicates that the work has been completed as shown in the medical examination or the blood test of number 1. States of "incomplete" include "not started" indicating that the work has not started, "working" indicating that the work is under progress, and "interruption" indicating that the work has been suspended due to a certain trouble or the like.

In the case of a medical examination, admission reservation, an anesthesia request, a surgery application, an anesthesia consent form, an operation consent form, or the like, when a medical staff member inputs that the work has been completed through the electronic medical record 18, the work information management unit 45 changes the progress to "complete" from "incomplete". In the case of a subject examination, such as a blood test or urinalysis, when the examination value is recorded in the medical record DB 14A, the work information management unit 45 changes the progress to "complete" from "incomplete". In the case of an image examination, such as an electrocardiogram examination, a CT examination, and a DR examination, when the examination image 19 is recorded in the image DB 15A or when the medical report 20 is recorded in the report DB 16A, the work information management unit 45 changes the progress to "complete" from "incomplete".

Work information is recorded for a work item that has been determined to be performed by making a medical appointment or an admission reservation or by issuing an order. On the other hand, no work information is recorded in the work item for which there is no work schedule, as shown by a dotted frame and a reference numeral BL of the blood test of number 4.

In Fig. 9, the work list 50 is displayed on the display screen 21. The work list 50 is configured to include a patient array axis Y on which patient information of each patient including patient identification information is arranged, an item array axis X on which work items are arranged, and a plurality of cells 51 that are intersections between patient information and work items. The patient array axis Y is disposed in the column direction, and the item array axis X is disposed in the row direction.

On the left end of the work list 50, a patient column 52 is provided along the patient array axis Y. On the upper end of the work list 50, an item column 53 is provided along the item array axis X. In the patient column 52, item numbers and patient information are displayed side by side in the item number order along the patient array axis Y. In the item column 53, work items are displayed side by side along the item array axis X. Work items of the item column 53 are arranged from left to right in order in which the work items are usually performed. For example, a medical examination is usually the work performed first. Accordingly, work items of the medical examination are arranged on the left end. In addition, an admission reservation or surgery application is the work performed according to the examination result after performing a subject examination or an image examination. Accordingly, the admission reservation or the surgery application is disposed on the right side of the work item of the subject examination or the image examination.

Each cell 51 has a rectangular shape in which a side along the item array axis X is slightly longer than a side along the patient array axis Y. The cells 51 include an information display cell 51A in which work information is displayed and a blank cell 51B in which no work information is displayed. The information display cell 51 A is displayed at a position corresponding to a work item, which is determined to be performed and in which work information is recorded, in the work information table 22. On the other hand, as shown by the reference numeral BL in Fig. 8, the blank cell 51 B is displayed at a position corresponding to a work item, for which work is not scheduled to be performed and in which no work information is recorded, in the work information table 22.

In the information display cell 51A, it is possible to perform spatially aggregating display using the aggregating display function. In the case of aggregating display, the blank cells 51B are deleted, and the information display cells 51A are packed together in either the row direction (direction of the item array axis X) or the column direction (direction of the patient array axis Y).

Thus, the work list 50 has the following three display modes. That is, these are a first display mode in which the blank cells 51 B are displayed without being deleted, a second display mode in which the blank cells 51B are deleted so that the information display cells 51 A are aggregatively displayed by being packed together in the row direction, and a third display mode in which the blank cells 51 B are deleted so that the information display cells 51A are aggregatively displayed by being packed together in the column direction. The display mode switching function is a function for switching among the three display modes.

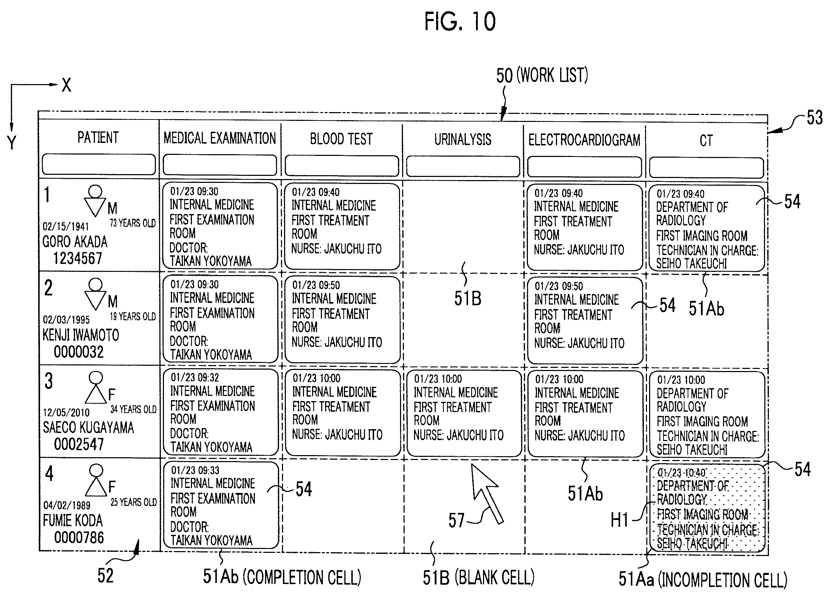

An icon 54 is disposed in the information display cell 51 A. Characters of a plurality of rows showing the specific content of work information is displayed in the icon 54. The characters displayed in the icon 54 are the content through which the medical staff can sufficiently understand the work information. For example, date and time on which a medical examination is reserved or has been performed, a medical department, a room, and a doctor in charge are displayed in the icon 54 disposed in the work item of the medical examination (also refer to Fig. 10). On the other hand, the icon 54 is not disposed in the blank cell 51 B, but is blank literally.

In the icon 54, the display color of chromatic color is set in advance for each work item. For example, the icon 54 disposed in the work item of medical examination is russet brow, and the icon 54 disposed in the work item of blood test is yellow ocher. Characters displayed in the icon 54 are displayed in white.

In addition to the work list 50, a display mode switching button 55, a vertical scroll bar 56A, a horizontal scroll bar 56B, and the like are displayed on the display screen 21. The display mode switching button 55 and the scroll bars 56A and 56B can be operated with a cursor 57.

The display mode switching button 55 is a toggle type button for inputting an instruction for sequential switching of three display modes of the work list 50 through the same input operation, and is provided on the right side of the work list 50. When the display mode switching button 55 is selected by the cursor 57, a request to edit the display screen 21 is transmitted to the medical assistance server 12 from the client terminal 11. In addition, on the display screen 21 that is distributed first in response to the distribution request, the work list 50 is displayed in the first display mode in which the blank cells 51B are not deleted as shown in Fig. 9.

The vertical scroll bar 56A is provided along the patient array axis Y on the right end of the display screen 21 opposite the patient column 52. The horizontal scroll bar 56B is provided along the item array axis X on the lower end of the display screen 21 opposite the item column 53. The vertical scroll bar 56A is displayed in a case where some of the patient information of the patient column 52 are not displayed on the display screen 21, that is, some of the patient information of the patient column 52 do not fit in the display screen 21 at one time. The horizontal scroll bar 56B is displayed in a case where some of the work items of the item column 53 are not displayed on the display screen 21, that is, some of the work items of the item column 53 do not fit in the display screen 21 at one time. The non-display portion can be displayed by a scroll operation, such as the operation of the cursor 57 on each of the scroll bars 56A and 56B or the rotation of a wheel button of a mouse.

As shown in Fig. 10 obtained by enlarging a part of the work list 50, the information display cells 51 A are divided into two types according to the progress. That is, the two types of information display cells 51A are an incompletion cell 51Aa in which the progress is "incomplete" and a completion cell 51 Ab in which the progress is "complete".

With the classification of the information display cell 51A according to the progress, the icons 54 are displayed so as to be distinguished in the incompletion cell 51Aa and the completion cell 51Ab. Specifically, in the incompletion cell 51Aa, as shown by hatching H1, the icon 54 is displayed in achromatic color, such as gray, instead of the display color of chromatic color set in advance. On the other hand, in the completion cell 51 Ab, the icon 54 is displayed in the display color of chromatic color set in advance. In addition to or instead of the change of the display color, characters of work information may be displayed in bold in the icon 54 of the completion cell 51Ab and characters of work information may be displayed in small characters or dotted characters in the icon 54 of the incompletion cell 51Aa, so that the incompletion cell 51Aa and the completion cell 51Ab are displayed so as to be distinguished from each other.

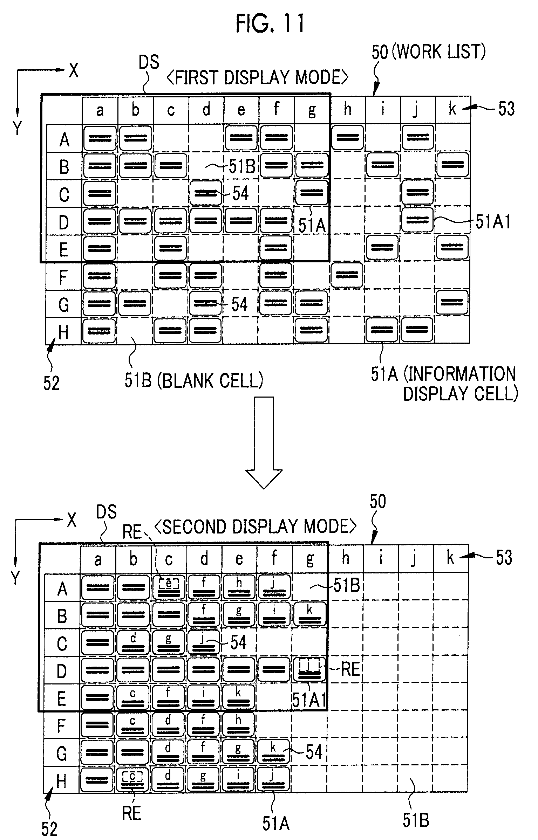

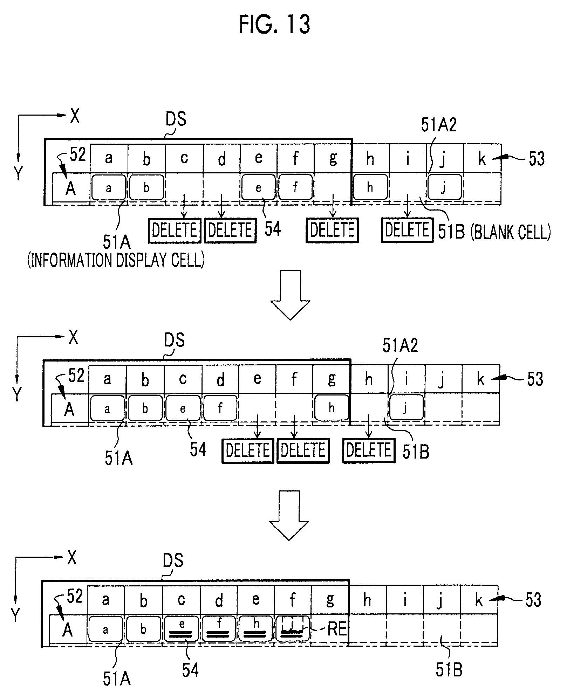

Fig. 11 shows the work list 50 of the first display mode and the work list 50 of the second display mode. In Fig. 11, in order to avoid complication, explanation will be given using the simple work list 50 in which the characters of the work information of the icon 54 are expressed as lines, the information display cell 51A is displayed without distinction between the incompletion cell 51 Aa and the completion cell 51 Ab, the patient information of the patient column 52 is expressed in uppercase letters of A, B, C, ..., and the work items of the item column 53 are expressed in lowercase letters of a, b, c, .... In addition, a display portion of the display screen 21 is shown by a solid thick frame and a reference numeral DS. The same is true for subsequent Figs. 13, 14, 16, and the like.

In Fig. 11, the upper portion shows the work list 50 of the first display mode, and the lower portion shows the work list 50 of the second display mode. In the first display mode, the blank cells 51 B are not deleted, and the information display cells 51 A are displayed without being packed together. On the other hand, in the second display mode, the blank cells 51B are deleted, and the information display cells 51A are aggregatively displayed by being packed together in the row direction.

In the second display mode, as shown by a dotted frame and a reference numeral RE, a work item corresponding to the case of the original position is displayed in the information display cell 51 A shifted from the original position in the first display mode due to aggregating display. For example, an information display cell 51A1 located at the position of a work item j and patient information D in the first display mode is shifted to the position of the work item g in the second display mode. Therefore, the work item j corresponding to the case of the original position is displayed in the icon 54.

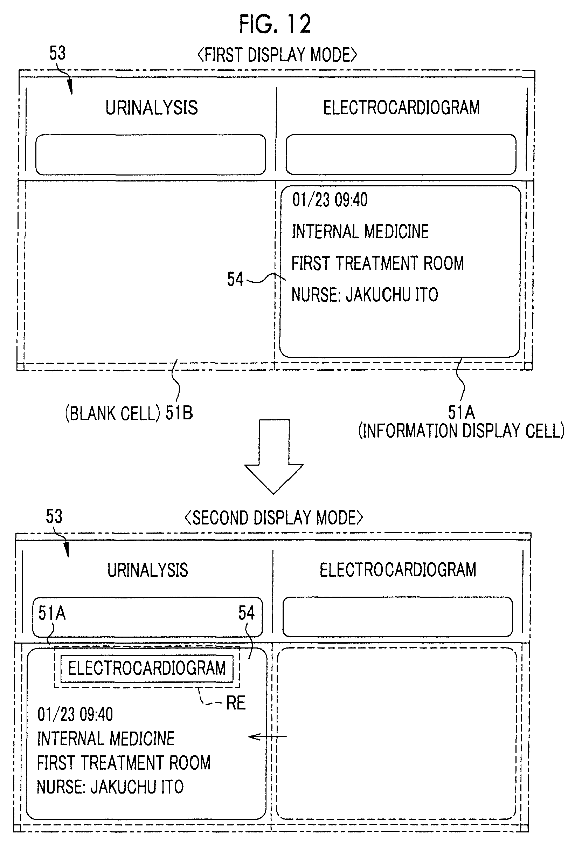

More specifically, as shown in Fig. 12, in a case where the information display cell 51A located at the position of the work item of electrocardiogram examination in the first display mode is shifted to the position of the work item of urinalysis in the second display mode, "electrocardiogram" is displayed in the icon 54 of the information display cell 51A.

In Fig. 13, the upper portion shows the row of patient information A in the first display mode in the upper portion of Fig. 11. In this stage, among all of the cells 51 that form the row of the patient information A, the cells 51 corresponding to the work items a, b, e, f, h, and j are the information display cells 51A, and the cells 51 corresponding to the work items c, d, g, i, and k are the blank cells 51 B. The screen display control unit 43 deletes the blank cells 51B corresponding to the work items c, d, g, and i that are present between the information display cells 51 A, among the blank cells 51 B corresponding to the work items c, d, g, i, and k, using the aggregating display function. In addition, as shown in the middle, the information display cell 51A located on the right side of the deleted blank cell 51B is shifted to the deletion position of the blank cell 51B. For example, an information display cell 51 A2 located at the position of the work item j in the upper portion is shifted to the position of the work item i where the blank cell 51 B has been deleted.

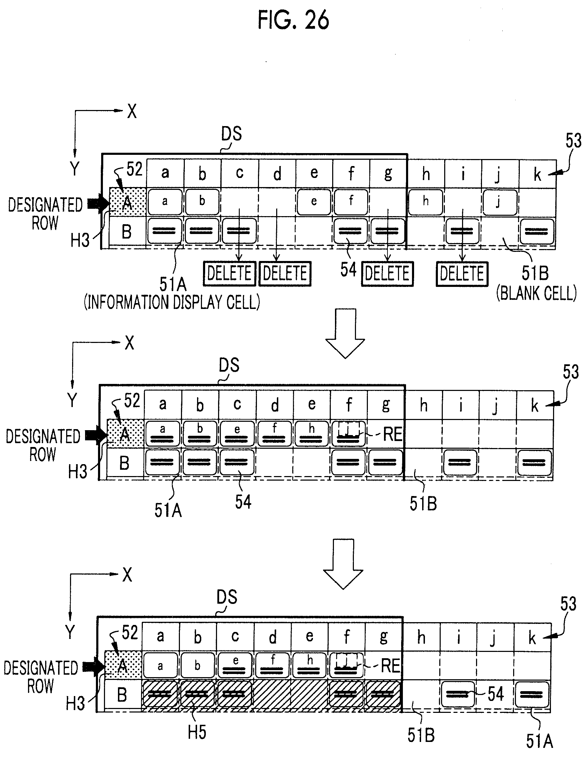

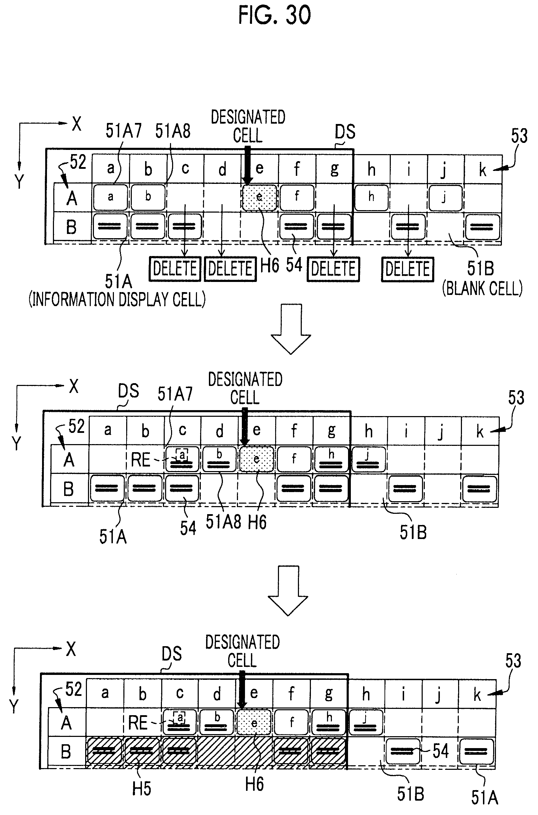

The screen display control unit 43 packs the information display cells 51A in the row direction by performing the deletion of the blank cell 51B and shift of the information display cell 51A to the left side, and finally collects the information display cells 51A on the left side (side of the patient column 52) as shown in the lower portion, thereby realizing the second display mode. In other words, the screen display control unit 43 moves the information display cell 51A to the position of the blank cell 51B in the row direction until no blank cell 51B is present between the information display cells 51A. In Fig. 13, for convenience of explanation, lowercase letters showing the work items corresponding to the case of the original position are displayed in the icons 54 of all of the information display cells 51A in the upper, middle, and lower portions. The same is true for Figs. 26, 30, and the like.

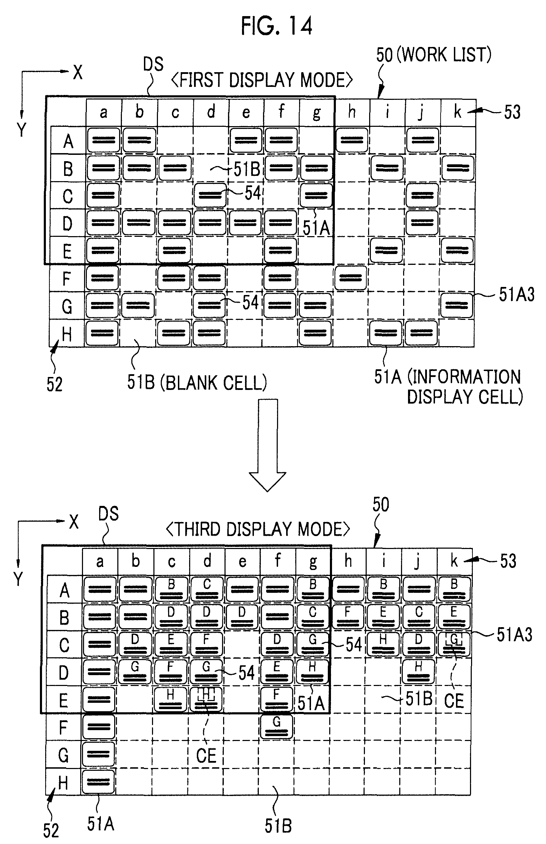

In Fig. 14, the upper portion shows the work list 50 of the first display mode as in Fig. 11, and the lower portion shows the work list 50 of the third display mode. In the third display mode, the blank cells 51B are deleted, and the information display cells 51 A are aggregatively displayed by being packed together in the column direction.

In the third display mode, as shown by a dotted frame and a reference numeral CE, patient identification information corresponding to the case of the original position is displayed in the information display cell 51A shifted from the original position in the first display mode due to aggregating display. For example, an information display cell 51A3 located at the position of a work item k and patient information G in the first display mode is shifted to the position of patient information C in the third display mode. Therefore, patient identification information of the patient information G corresponding to the case of the original position is displayed in the icon 54.

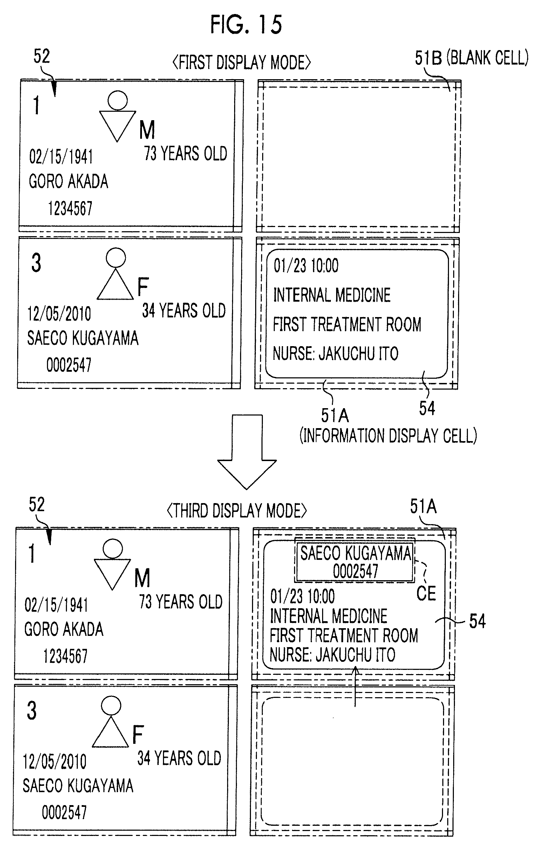

More specifically, as shown in Fig. 15, in a case where the information display cell 51A located at the position of patient name "Kugayama Saeco" and patient ID "0002547" in the first display mode is shifted to the position of patient name "Goro Akada" and patient ID "1234567" in the third display mode, patient name "Kugayama Saeco" and patient ID "0002547" are displayed in the icon 54 of the information display cell 51 A.

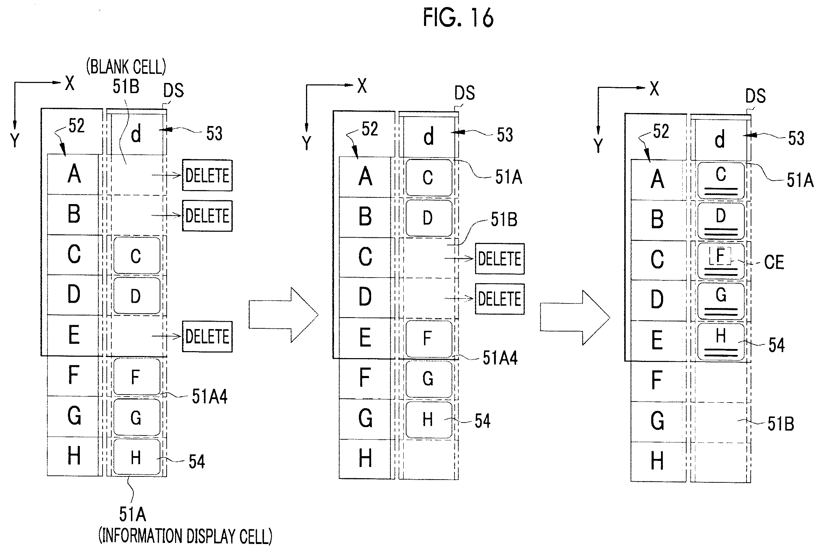

The left side of Fig. 16 shows a column of the work item d in the first display mode in the upper portion of Fig. 14. In this stage, among all of the cells 51 that form the column of the work item d, the cells 51 corresponding to patient information C, D, F, G, and H are the information display cells 51 A, and the cells 51 corresponding to patient information A, B, and E are the blank cells 51B. The screen display control unit 43 deletes the blank cells 51 B corresponding to the patient information A, B, and E, which are present between the information display cells 51 A, using the aggregating display function. In addition, as shown in the middle, the information display cell 51A located on the lower side of the deleted blank cell 51 B is shifted to the deletion position of the blank cell 51 B. For example, an information display cell 51A4 located at the position of the patient information F on the left side is shifted to the position of the patient information E where the blank cell 51B has been deleted.

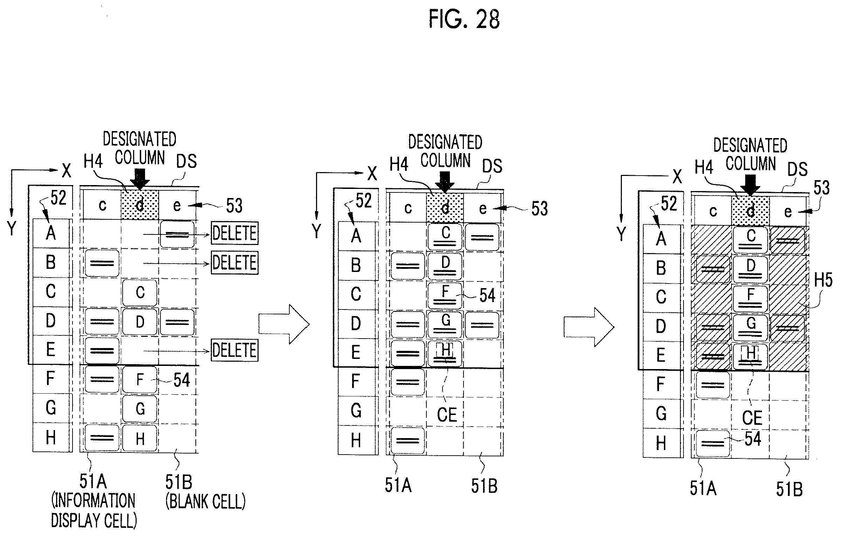

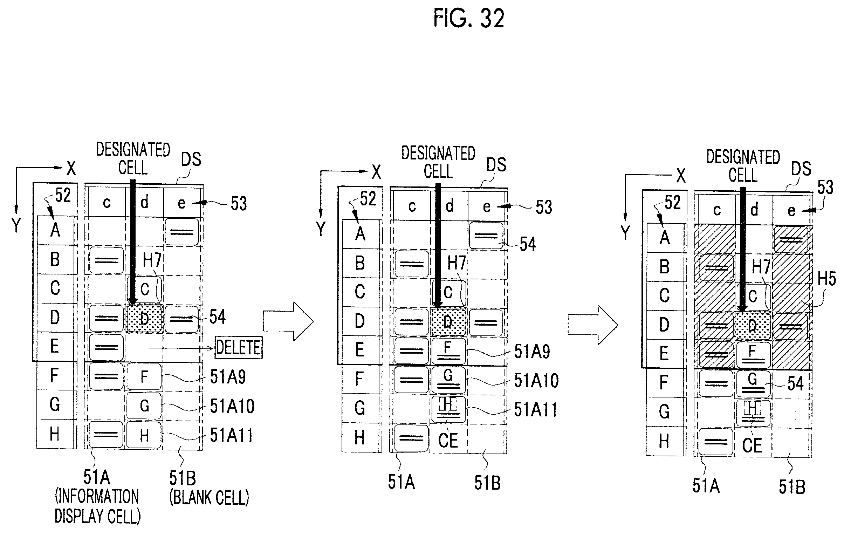

The screen display control unit 43 packs the information display cells 51 A in the column direction by performing the deletion of the blank cell 51 B and shift of the information display cell 51A to the upper side, and finally collects the information display cells 51A on the upper side (side of the item column 53) as shown on the right side, thereby realizing the third display mode. In other words, the screen display control unit 43 moves the information display cell 51A to the position of the blank cell 51B in the column direction until no blank cell 51B is present between the information display cells 51A. In Fig. 16, as in the case of Fig. 13, for convenience of explanation, uppercase letters showing the patient information corresponding to the case of the original position are displayed in the icons 54 of all of the information display cells 51A on the left, middle, and right sides. The same is true for Figs. 28, 32, and the like.

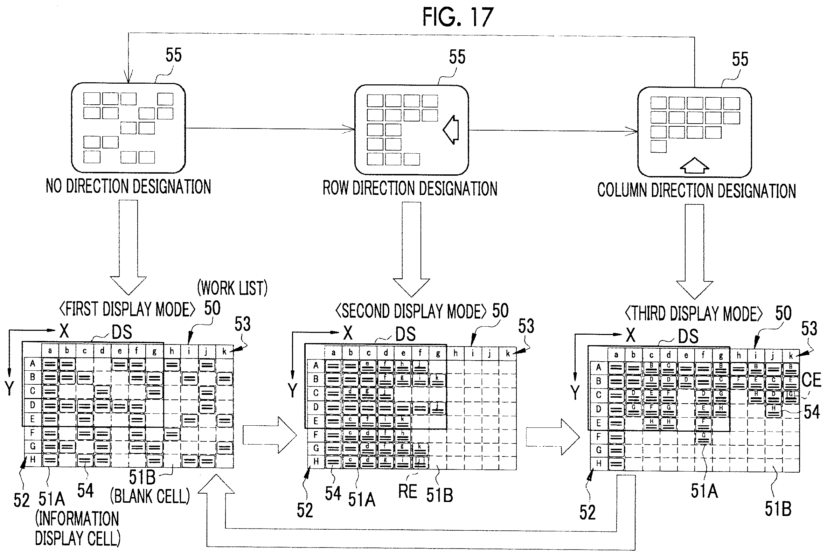

In Fig. 17, whenever the display mode switching button 55 is selected by the cursor 57, the display mode switching button 55 is sequentially switched among three instruction states of no direction designation shown on the upper left side, row direction designation shown in the upper middle, and column direction designation shown on the upper right side. More specifically, the display mode switching button 55 transitions to the state of row direction designation in a case where the display mode switching button 55 is selected in the state of no direction designation, and transitions to the state of column direction designation in a case where the display mode switching button 55 is selected in the state of row direction designation. Then, in a case where the display mode switching button 55 is selected in the state of column direction designation, the display mode switching button 55 returns to the state of no direction designation.

In a case where the display mode switching button 55 transitions from the state of no direction designation to the state of row direction designation, the browser control unit 36 issues an editing request to designate the row direction as a direction in which the information display cells 51A are packed together. The editing request is received by the request receiving unit 41, and is output to the screen display control unit 43. The screen display control unit 43 switches the display mode of the work list 50 from the first display mode to the second display mode using the display mode switching function. That is, in a case where the display mode switching button 55 is in the state of row direction designation, the display mode of the work list 50 is switched to the second display mode as shown in the middle of the lower portion.

In a case where the display mode switching button 55 transitions from the state of row direction designation to the state of column direction designation, the browser control unit 36 issues an editing request to designate the column direction as a direction in which the information display cells 51 A are packed together. The editing request is received by the request receiving unit 41, and is output to the screen display control unit 43. The screen display control unit 43 switches the display mode of the work list 50 from the second display mode to the third display mode using the display mode switching function. That is, in a case where the display mode switching button 55 is in the state of column direction designation, the display mode of the work list 50 is switched to the third display mode as shown on the lower right side.

In a case where the display mode switching button 55 transitions from the state of column direction designation to the state of no direction designation, the browser control unit 36 issues an editing request to display the information display cells 51A, which are not packed together, without deleting the blank cell 51B. The editing request is received by the request receiving unit 41, and is output to the screen display control unit 43. The screen display control unit 43 switches the display mode of the work list 50 from the third display mode to the first display mode using the display mode switching function. That is, in a case where the display mode switching button 55 is in the state of no direction designation, the display mode of the work list 50 is switched to the first display mode as shown on the lower left side.

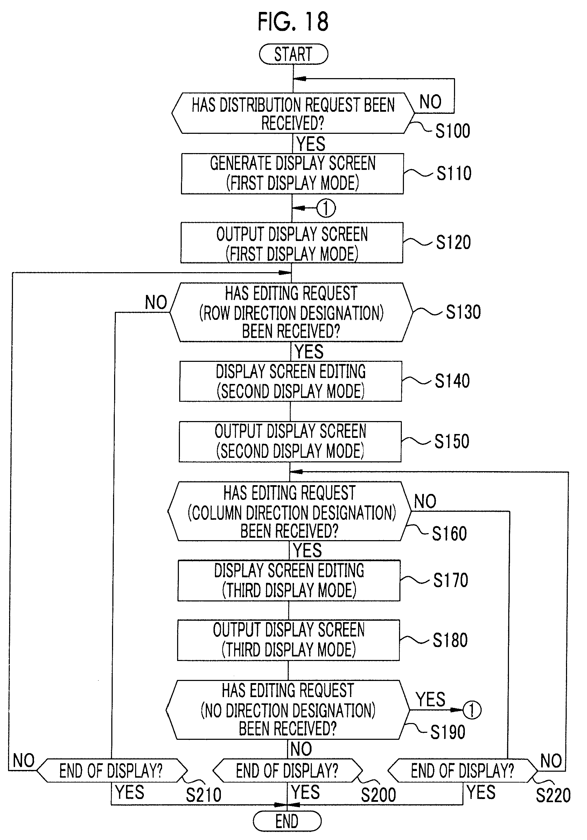

Hereinafter, the operation of the above configuration will be described with reference to the flowchart shown in Fig. 18. First, a medical staff member performs authentication by accessing the medical assistance server 12 through the client terminal 11. After the authentication, a distribution instruction screen is displayed on the display 29A of the client terminal 11. The medical staff member inputs a distribution instruction through the distribution instruction screen. Then, a distribution request of the display screen 21 is transmitted to the medical assistance server 12 from the client terminal 11.

In the medical assistance server 12, the distribution request is received by the request receiving unit 41 (YES in step S100). The distribution request is output to the screen generation unit 42. Then, work information is output to the screen generation unit 42 from the work information management unit 45. Then, as shown in step S110, the display screen 21 based on the work information is generated by the screen generation unit 42. The generated display screen 21 is output to the client terminal 11, which is a source of the distribution request, by the distribution control unit 44 (step S120). In this case, the display mode of the work list 50 of the display screen 21 is the first display mode shown in Fig. 9.

In the client terminal 11, the display screen 21 from the medical assistance server 12 is received by the browser control unit 36. Then, the display screen 21 is displayed on the display 29A by the GUI control unit 35.

The medical staff member views the display screen 21, and selects the display mode switching button 55 with the cursor 57 when necessary. Then, an editing request of the display screen 21 is transmitted to the medical assistance server 12 from the client terminal 11. In the medical assistance server 12, the editing request is received by the request receiving unit 41. The editing request is output to the screen display control unit 43.

In a case where the display mode switching button 55 is selected when the display mode of the work list 50 is the first display mode and the display mode switching button 55 transitions from the state of no direction designation to the state of row direction designation, the request receiving unit 41 receives an editing request to designate the row direction as a direction in which the information display cells 51A are packed together (YES in step S130).

In this case, the information display cells 51A are packed together in the row direction by the aggregating display function and the display mode switching function of the screen display control unit 43, so that the display mode of the work list 50 is switched from the first display mode to the second display mode (step S140). At this time, in the information display cell 51 A shifted from the original position in the first display mode due to aggregating display, the work item RE corresponding to the case of the original position is displayed. The display screen 21 on which the display mode of the work list 50 has been switched to the second display mode is output to the client terminal 11, which is a source of the editing request, by the distribution control unit 44 (step S150).

In a case where the display mode switching button 55 is selected when the display mode of the work list 50 is the second display mode and the display mode switching button 55 transitions from the state of row direction designation to the state of column direction designation, the request receiving unit 41 receives an editing request to designate the column direction as a direction in which the information display cells 51 A are packed together (YES in step S160).

In this case, the information display cells 51A are packed together in the column direction by the aggregating display function and the display mode switching function of the screen display control unit 43, so that the display mode of the work list 50 is switched from the second display mode to the third display mode (step S170). At this time, in the information display cell 51A shifted from the original position in the first display mode due to aggregating display, the patient identification information CE corresponding to the case of the original position is displayed. The display screen 21 on which the display mode of the work list 50 has been switched to the third display mode is output to the client terminal 11, which is a source of the editing request, by the distribution control unit 44 (step S 180).

In a case where the display mode switching button 55 is selected when the display mode of the work list 50 is the third display mode and the display mode switching button 55 transitions from the state of column direction designation to the state of no direction designation, the request receiving unit 41 receives an editing request to display the information display cells 51 A, which are not packed together, without deleting the blank cell 51 B (YES in step S190).

In this case, the process returns to step S120 in which the display screen 21, on which the display mode of the work list 50 is the first display mode, is output to the client terminal 11, which is a source of the editing request, by the distribution control unit 44. The process of steps S120 to S190 is continued until the end of the display of the display screen 21 (YES in steps S200, S210, and S220).

In the first display mode, the blank cells 51B are not deleted and the information display cells 51 A are not packed together, so that the information display cells 51 A and the blank cells 51B are displayed so as to be scattered. Therefore, since it is possible to grasp to which patient identification information and work item the information display cell 51 A corresponds depending on the position of the information display cell 51 A, it is possible to take a look at the general overview of the work information.

On the other hand, in the second and third display modes, the blank cells 51 Bare deleted, and the information display cells 51 A are packed together in the row direction or in the column direction. Accordingly, the information display cell 51 A that was not located in the display portion DS in the first display mode, such as the information display cell 51 A2 located at the position of the work item j in the upper portion of Fig. 13 or the information display cell 51A4 located at the position of the patient information F on the left side of Fig. 16, can be located in the display portion DS in the second and third display modes. As a result, since the degree of at-a-glance listing of the work list 50 is improved, it is possible to check a larger amount of work information at one time.

In addition, in the second display mode, the information display cells 51A are packed together in the row direction perpendicular to the patient array axis Y. Therefore, it is possible to check how much work is to be performed on each patient. On the other hand, in the third display mode, the information display cells 51A are packed together in the column direction perpendicular to the item array axis X. Therefore, it is possible to check how much work is to be performed.

In addition, since the switching among the first to third display modes having unique features is possible, it is possible to display the work list 50 of the display mode that each medical staff member desires according to various purposes.

In addition, in the second and third display modes, the work item RE or the patient identification information CE corresponding to the case of the original position is displayed in the information display cell 51A shifted from the original position in the first display mode due to aggregating display. Therefore, also in the second and third display modes, it is possible to grasp to which work item or patient identification information the information display cell 51A corresponds, as in the first display mode.

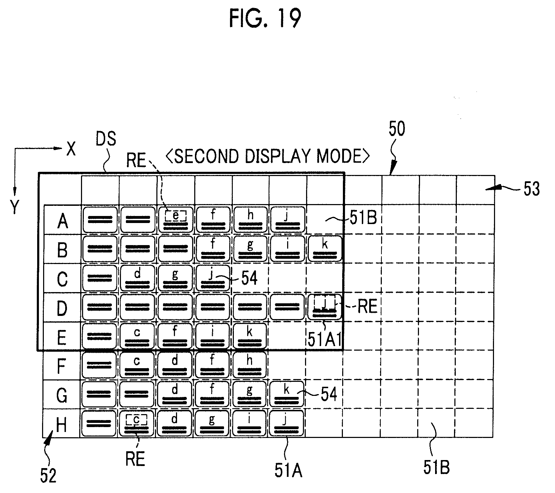

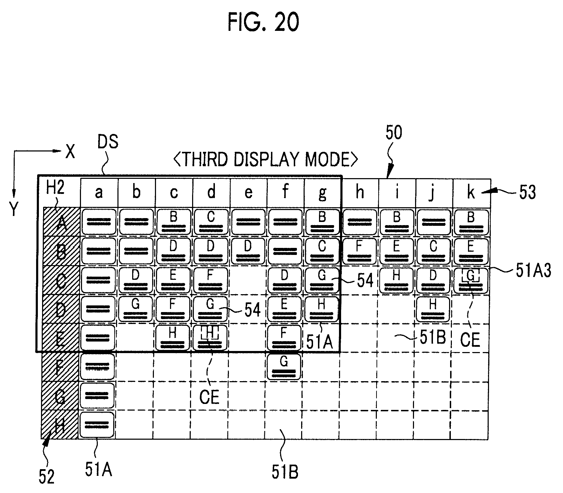

In the case of displaying the work item RE or the patient identification information CE, displaying the original work item or the original patient information in the item column 53 or the patient column 52 causes confusion. Therefore, in the case of displaying the work item RE or the patient identification information CE, it is preferable to take measures, such as not displaying the work item of the item column 53 as shown in Fig. 19 or making the patient information of the patient column 52 inconspicuous through gray-out processing as shown by hatching H2 in Fig. 20.

Since the display mode of the work list 50 is sequentially switched by the toggle type display mode switching button 55, the display mode of the work list 50 can be switched easily and smoothly. Therefore, it is possible to check the work information efficiently.

In addition, the sequential switching of the display mode of the work list 50 may not depend on the toggle type display mode switching button 55. For example, a button for switching between enabling and disabling of the aggregating display function is provided on the display screen 21. In a case where the aggregating display function is enabled by the button, the display mode of the work list 50 is sequentially switched among the first to third display modes by an operation of rotating a wheel button of a mouse or by mouse gestures such as dragging to the right side in a state in which a mouse is right-clicked. In the case of the operation of rotating the wheel button of the mouse, the display mode of the work list 50 is switched, for example, whenever the wheel button is half rotated in the same direction. In this case, the operation of half rotating the wheel button in the same direction corresponds to the same input operation. In the case of mouse gestures, the display mode of the work list 50 is switched whenever a mouse gesture is performed. In this case, the mouse gesture corresponds to the same input operation.

In addition, the display mode of the work list 50 may not be sequentially switched. For example, three display mode switching buttons corresponding to the display mode switching button 55 of the three designated states shown in Fig. 17 may be provided on the display screen 21, and the display mode of the work list 50 may be switched in response to the selection of each display mode switching button by the cursor 57. Alternatively, a case where certain patient information of the patient column 52 is selected may be regarded as the row direction designation, and a case where a certain work item of the item column 53 is selected may be regarded as the column direction designation. That is, the patient information of the patient column 52 and the work item of the item column 53 may be made to function as display mode switching buttons without providing a dedicated display mode switching button.

The progress of work having "incomplete" and "complete" is included in work information, the incompletion cell 51Aa and the completion cell 51 Ab are provided as two types of information display cell 51A, and the icon 54 is displayed so as to be distinguished between the incompletion cell 51Aa and the completion cell 51Ab. Accordingly, the progress of each work piece can be seen at a glance. In addition, since the number of incomplete work pieces can also be seen at a glance by the number of incompletion cells 51Aa, a medical staff member can check how many incomplete work pieces, among work pieces that the medical staff member is in charge of, are present.

### [Second embodiment]

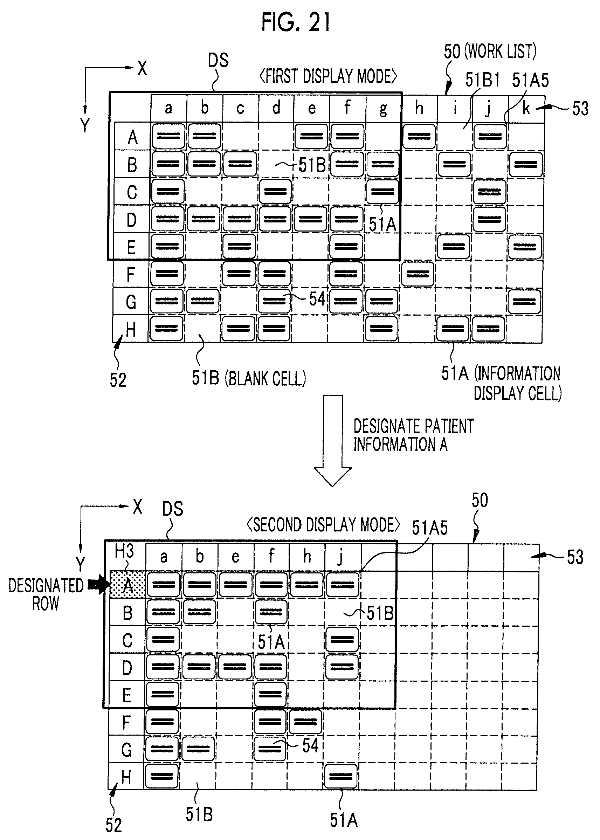

In the first embodiment described above, the aggregating display is performed for all of a plurality of rows or columns of the work list 50. In a second embodiment shown in Figs. 21 to 24, however, the aggregating display is performed for one of the plurality of rows of the work list 50 or one of the plurality of columns of the work list 50.

In the present embodiment, it is possible to select each piece of patient information of the patient column 52 and each work item of the item column 53 with the cursor 57. The selection of patient information corresponds to the designation of one of the plurality of rows of the work list 50, and the selection of a work item corresponds to the designation of one of the plurality of columns of the work list 50. In addition, in the present embodiment, a button for switching between enabling and disabling of the aggregating display function is provided on the display screen 21.

In a case where the aggregating display function is enabled and patient information is selected, the browser control unit 36 issues an editing request to pack the information display cells 51A in the row of the selected patient information (hereinafter, referred to as a designated row) in the row direction. That is, not only the designation of a row but also the designation in the row direction is included in the editing request in a case where patient information is selected. The editing request is received by the request receiving unit 41, and is output to the screen display control unit 43. The screen display control unit 43 switches the display mode of the work list 50 to the second display mode.

On the other hand, in a case where the aggregating display function is enabled and a work item is selected, the browser control unit 36 issues an editing request to pack the information display cells 51 A in the column of the selected work item (hereinafter, referred to as a designated column) in the column direction. That is, not only the designation of a work item but also the designation in the column direction is included in the editing request in a case where a work item is selected. The editing request is received by the request receiving unit 41, and is output to the screen display control unit 43. The screen display control unit 43 switches the display mode of the work list 50 to the third display mode.

In Fig. 21, as in Fig. 11 and the like, the upper portion shows the work list 50 of the first display mode, and the lower portion shows the work list 50 of the second display mode. For the work list 50 of the second display mode, a case is illustrated in which the patient information A is selected as a designated row as shown by hatching H3. In the second display mode, the blank cells 51 B in the designated row are deleted, and the information display cells 51 A in the designated row are aggregatively displayed by being packed together in the row direction. For example, a blank cell 51B located at the position of the work item i and the patient information A in the first display mode is deleted, and an information display cell 51A5 located at the position of the work item j and the patient information A in the first display mode is packed at the position of the work item f in the first display mode in the second display mode.

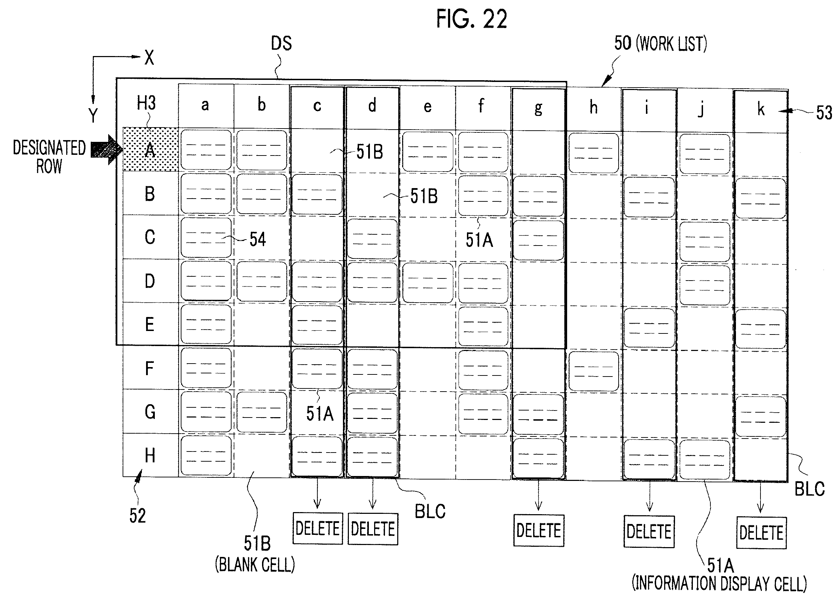

In addition, as shown by a solid thick frame and a reference numeral BLC in Fig. 22, for a column in which the blank cell 51 B is present in the designated row, the entire column is deleted regardless of the presence or absence of the information display cell 51A. For example, in the column of the work item c, the information display cell 51 A is located at the position of patient information B, D, E, F, and H. However, since the position of the patient information A that is a designated row is the blank cell 51B, the column of the work item c is deleted. In addition, in the column of the work item k, the information display cell 51A is located at the position of patient information B, E, and G. In addition, the column of the work item k is a final column. However, since the position of the patient information A is the blank cell 51 B, the column of the work item k is deleted. Therefore, in the second display mode shown in Fig. 21, only the work item of a column in which the information display cell 51A is present in the designated row is displayed in the item column 53. Specifically, columns in which the blank cell 51B is present in the designated row are columns of the work items c, d, g, i, and k. Specifically, columns in which the information display cell 51A is present in the designated row are columns of the work items a, b, e, f, h, and j.

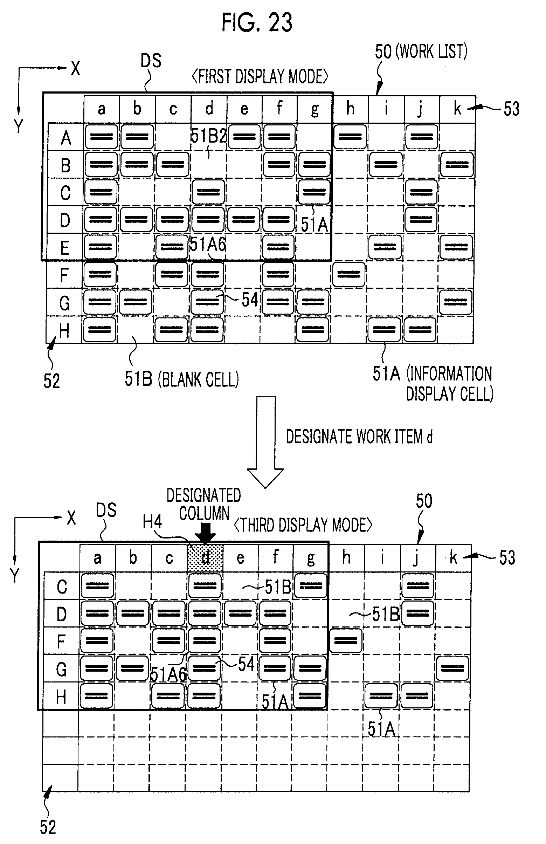

On the other hand, Fig. 23 shows the work list 50 of the third display mode in the lower portion. For the work list 50 of the third display mode, a case is illustrated in which the work item d is selected as a designated column as shown by hatching H4. In the third display mode, the blank cells 51B in the designated column are deleted, and the information display cells 51A in the designated column are aggregatively displayed by being packed together in the column direction. For example, a blank cell 51B2 located at the position of the work item d and the patient information B in the first display mode is deleted, and an information display cell 51A6 located at the position of the work item d and the patient information F in the first display mode is packed at the position of the patient information C in the first display mode in the second display mode.

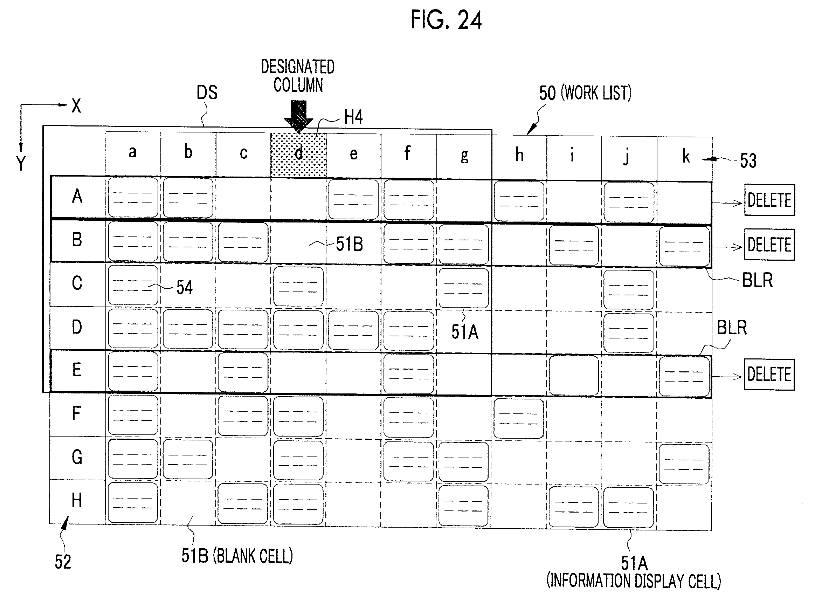

In addition, as shown by a solid thick frame and a reference numeral BLR in Fig. 24, for a row in which the blank cell 51B is present in the designated column, the entire row is deleted regardless of the presence or absence of the information display cell 51A. For example, in the row of the patient information A, the information display cell 51 A is located at the positions of work items a, b, e, f, h, and j. However, since the position of the work item d that is a designated column is the blank cell 51B, the row of the patient information A is deleted. Therefore, in the third display mode shown in Fig. 23, only the patient information of a row in which the information display cell 51 A is present in the designated column is displayed in the patient column 52. Specifically, rows in which the blank cell 51B is present in the designated column are rows of the patient information A, B, and E. Specifically, rows in which the information display cell 51 A is present in the designated column are rows of the patient information C, D, F, G, and H.

Thus, since the aggregating display is performed for a designated row or a designated column, the degree of at-a-glance listing in the designated row or the designated column is improved. Therefore, it is possible to check the work information focused on each patient or to check the work information focused on each work item. More specifically, by switching the display mode of the work list 50 to the second display mode by selecting the patient information of an examination target patient at the time of a medical examination, it is possible to check the work information of the work for the examination target patient or the number of work pieces. Alternatively, by switching the display mode of the work list 50 to the third display mode by selecting the work item of work that a medical staff member is in charge of at the start or end of the medical facility, it is possible to check the work information of the work that the medical staff member is in charge of or the number of work pieces.

In addition, since the entire row in which the blank cell 51B in the designated column is present is deleted in the second display mode and the entire column in which the blank cell 51B in the designated row is present is deleted in the third display mode. Therefore, since the number of rows or columns of the work list 50 is reduced, the work list 50 can be packed compactly. As a result, the display portion DS can be more effectively used.

In addition, in the second display mode, the position itself of a column in which the information display cell 51 A in the designated row is present is shifted in the row direction, but is shifted for each column. Accordingly, work items displayed in the item column 53 are not changed from the first display mode. For this reason, also in the second display mode, it is possible to grasp to which work item the information display cell 51A corresponds depending on the position of the information display cell 51A. In the present embodiment, therefore, there is no need to display the work item RE corresponding to the case of the original position of the first embodiment described above. This is the same for the third display mode. That is, since it is possible to grasp to which patient information the information display cell 51A corresponds depending on the position of the information display cell 51A, there is no need to display the patient identification information CE corresponding to the case of the original position of the first embodiment described above.

### [Third embodiment]

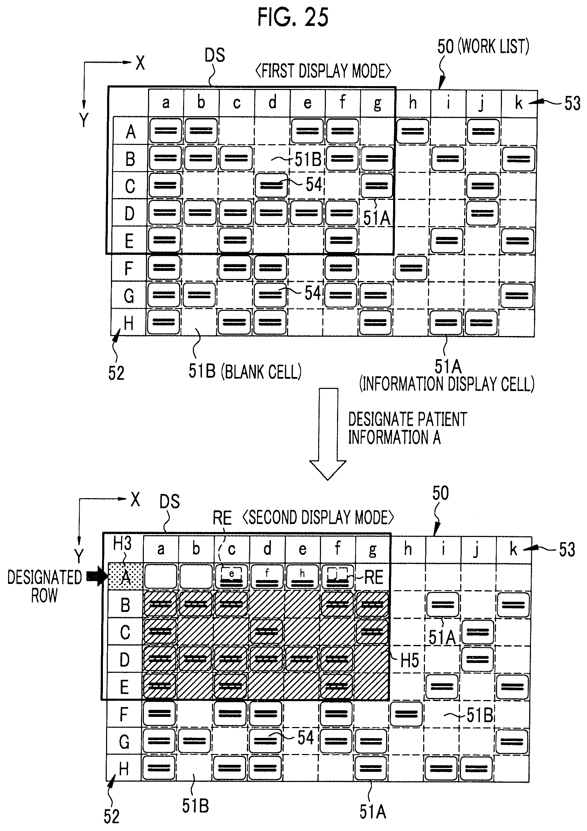

In a third embodiment shown in Figs. 25 to 28, aggregating display is performed for a designated row or a designated column as in the second embodiment described above, but the method of aggregating display is different from the second embodiment.

In Fig. 25, as in Fig. 11 and the like, the upper portion shows the work list 50 of the first display mode, and the lower portion shows the work list 50 of the second display mode. For the work list 50 of the second display mode, as in Fig. 21 in the second embodiment described above, a case is illustrated in which the patient information A is selected as a designated row as shown by hatching H3. Also in the present embodiment, similarly to the second embodiment described above, in the second display mode, the blank cells 51 B in the designated row are deleted, and the information display cells 51A in the designated row are aggregatively displayed by being packed together in the row direction.

In the second display mode, similarly to the first embodiment described above, the work item RE corresponding to the case of the original position is displayed in the information display cell 51 A shifted from the original position in the first display mode due to aggregating display.

In the present embodiment, however, aggregating display is not performed for rows other than the designated row. In addition, as shown by hatching H5, a designated row in which aggregating display is performed is highlighted by performing, for example, gray-out processing for covering rows, in which aggregating display is not performed, other than the designated row of the display portion DS with a gray shaded pattern.

As shown in the upper portion and the middle portion of Fig. 26, the screen display control unit 43 packs the information display cells 51A of the row of the patient information A in the row direction by performing the deletion of the blank cell 51B and shift of the information display cell 51A to the left side in the row of the patient information A that is a designated row using the aggregating display function, and finally collects the information display cells 51 A on the left side (side of the patient column 52), thereby realizing the second display mode. Then, as shown in the lower portion, gray-out processing is performed for rows other than the row of the patient information A.

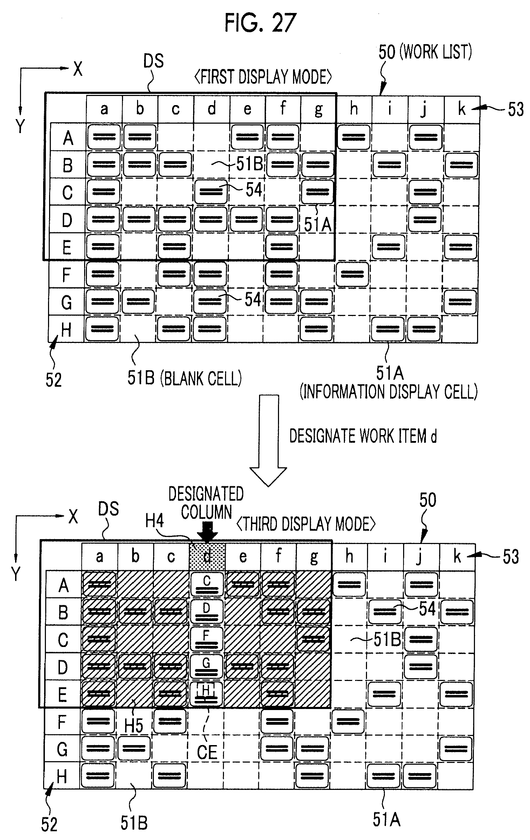

On the other hand, Fig. 27 shows the work list 50 of the third display mode in the lower portion. For the work list 50 of the third display mode, as in Fig. 23 in the second embodiment described above, a case is illustrated in which the work item d is selected as a designated column as shown by hatching H4. In the present embodiment, similarly to the second embodiment described above, in the third display mode, the blank cells 51 B in the designated column are deleted, and the information display cells 51A in the designated column are aggregatively displayed by being packed together in the column direction.

In the third display mode, as in the first embodiment described above, the patient identification information CE corresponding to the case of the original position is displayed in the information display cell 51A shifted from the original position in the first display mode due to aggregating display.

In the present embodiment, however, aggregating display is not performed for columns other than the designated column. In addition, as in the second display mode, as shown by hatching H5, a designated column is highlighted by performing gray-out processing for columns other than the designated column of display portion DS.

As shown on the left side and the middle of Fig. 28, the screen display control unit 43 packs the information display cells 51A of the column of the work item d in the column direction by performing the deletion of the blank cell 51B and shift of the information display cell 51A to the upper side in the column of the work item d that is a designated column using the aggregating display function, and finally collects the information display cells 51 A on the upper side (side of the item column 53), thereby realizing the third display mode. Then, as shown on the right side, gray-out processing is performed for columns other than the column of the work item d.

Also in the present embodiment, as in the second embodiment described above, the degree of at-a-glance listing in the designated row or the designated column is improved. Therefore, it is possible to check the work information focused on each patient or to check the work information focused on each work item.

In addition, since no aggregating display is performed for rows other than the designated row or columns other than the designated column, the display modes of the rows other than the designated row or the columns other than the designated column are not changed. For this reason, except for the designated row or the designated column, the position of the cell 51 is not changed before and after the switching of the display mode. Accordingly, compared with each of the embodiments described above in which the position of the cell 51 is changed before and after the switching of the display mode, no complicated impression is given to the medical staff.

In addition, since the designated row or the designated column is highlighted, it is possible to attract the attention of the medical staff to the designated row or the designated column.

As a method of highlighting the designated row or the designated column, other methods may be adopted. For example, the outlines of rows other than the designated row or columns other than the designated column may be displayed as thin lines, and the outline of the designated row or the designated column may be displayed as thick lines. Alternatively, a red color may be displayed as the background color of the designated row or the designated column, and a gray color may be displayed as the background color of the other rows or the other columns. In addition, the designated row or the designated column may be displayed so as to blink.

### [Fourth embodiment]

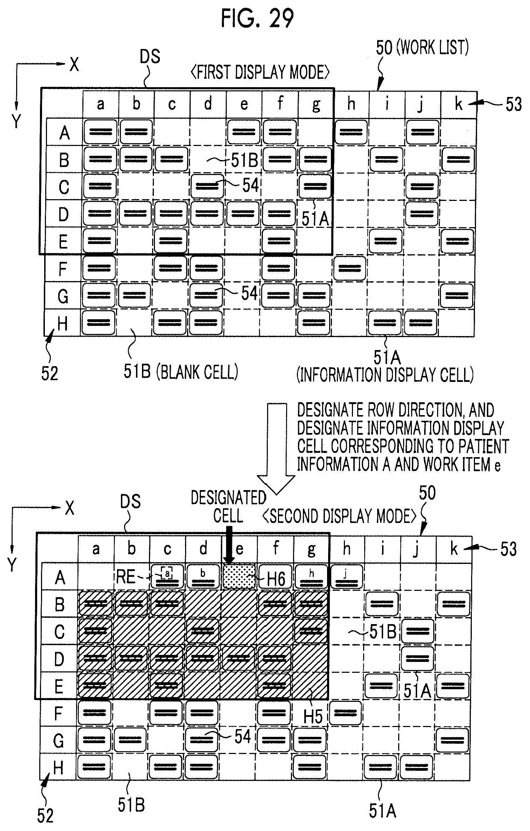

In each of the embodiments described above, the information display cells 51A are packed together on the left side or the upper side. In a fourth embodiment shown in Figs. 29 to 32, however, one information display cell 51A is located at the center and the other information display cells 51A are packed together on the left and right sides or upper and lower sides of the information display cell 51A located at the center. Therefore, in the present embodiment, it is possible to select the information display cell 51A with the cursor 57. The selection of the information display celt 51A corresponds to the designation of one cell 51 among a plurality of cells 51. In addition, in the present embodiment, a button for switching between enabling and disabling of the aggregating display function is provided on the display screen 21.

In this case, the designation of the row direction or the column direction is performed separately from the selection of the information display cell 51A, for example, by providing a row direction designation button and a column direction designation button on the display screen 21. Alternatively, as in the second and third embodiments described above, each piece of patient information of the patient column 52 and each work item of the item column 53 may be selectable by the cursor 57, and the selection of patient information may be regarded as the designation of the row direction and the selection of the work item may be regarded as the designation of the column direction.

In a case where the aggregating display function is enabled and the information display cell 51A is selected, the browser control unit 36 issues an editing request to pack the information display cells 51 A in a designated direction of the row direction and the column direction with the selected information display cell 51 A (hereinafter, referred to as a designated cell) being located at the center. The editing request is received by the request receiving unit 41, and is output to the screen display control unit 43. The screen display control unit 43 switches the display mode of the work list 50 to the second display mode or the third display mode.

In Fig. 29, as in Fig. 11 and the like, the upper portion shows the work list 50 of the first display mode, and the lower portion shows the work list 50 of the second display mode. For the work list 50 of the second display mode, a case is illustrated in which the row direction is designated and the information display cell 51A corresponding to the patient information A and the work item e is selected as a designated cell as shown by hatching H6. In the second display mode, the blank cells 51B in a row in which the designated cell is present (row of the patient information A) are deleted, and the information display cells 51A in the row in which the designated cell is present are aggregatively displayed by being packed together in the row direction with the designated cell being located at the center.

In addition, in the second display mode, as in the first and third embodiments described above, the work item RE corresponding to the case of the original position is displayed in the information display cell 51A shifted from the original position in the first display mode due to aggregating display.

In addition, as in the third embodiment described above, aggregating display is not performed for rows other than the row in which the designated cell is present, and gray-out processing is performed for the rows other than the row in which the designated cell is present as shown by hatching H5.

As shown in the upper portion of Fig. 30, the screen display control unit 43 deletes the blank cells 51 B located between the designated cell and the other information display cells 51A, among the blank cells 51 B in the row of the patient information A that is a row in which the designated cell is present, using the aggregating display function. Then, as shown in the middle, all of the information display cells 51 A in the row of the patient information A are shifted to the designated cell side. For example, information display cells 51A7 and 51A8 located at the positions of the work items a and b in the upper portion are shifted to the positions of the work items c and d on the right side that is the designated cell side.

The screen display control unit 43 packs the information display cells 51A in the row direction by performing the deletion of the blank cell 51 B and shift of the information display cell 51A to the designated cell side, and finally collects the information display cells 51A with the designated cell being located at the center as shown in the middle, thereby realizing the second display mode. Then, as shown in the lower portion, gray-out processing is performed for rows other than the row of the patient information A.

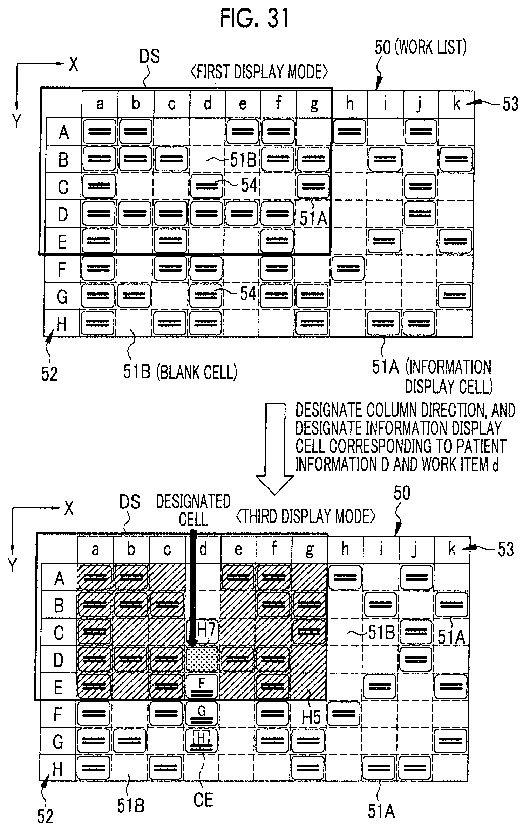

On the other hand, Fig. 31 shows the work list 50 of the third display mode in the lower portion. For the work list 50 of the third display mode, a case is illustrated in which the column direction is designated and the information display cell 51 A located at the position of the patient information D and the work item d is selected as a designated cell as shown by hatching H7. In the third display mode, the blank cells 51B in a column in which the designated cell is present (row of the work item d) are deleted, and the information display cells 51A in the column in which the designated cell is present are aggregatively displayed by being packed together in the column direction with the designated cell being located at the center.

In addition, in the third display mode, as in the first and third embodiments described above, the patient identification information CE corresponding to the case of the original position is displayed in the information display cell 51A shifted from the original position in the first display mode due to aggregating display.

In addition, as in the third embodiment described above, aggregating display is not performed for columns other than the column in which the designated cell is present, and gray-out processing is performed for the columns other than the column in which the designated cell is present as shown by hatching H5.

As shown on the left side of Fig. 32, the screen display control unit 43 deletes the blank cells 51 B located between the designated cell and the other information display cells 51 A, among the blank cells 51B in the column of the work item d that is a column in which the designated cell is present, using the aggregating display function. Then, as shown in the middle, all of the information display cells 51 A in the column of the work item d are shifted to the designated cell side. For example, information display cells 51 A9, 51A10, and 51A11 located at the positions of the patient information F, G, and H on the left side are shifted to the positions of the patient information E, F, and G on the upper side that is the designated cell side.

The screen display control unit 43 packs the information display cells 51A in the column direction by performing the deletion of the blank cell 51B and shift of the information display cell 51 A to the designated cell side, and finally collects the information display cells 51A with the designated cell being located at the center as shown in the middle, thereby realizing the third display mode. Then, as shown on the right side, gray-out processing is performed for columns other than the column of the work item d.

Thus, since the other information display cells 51A are aggregatively displayed with the designated cell being located at the center, it is possible to check the work information of the other information display cells 51 A with the designated cell as a reference. In addition, since the position of the designated cell is not changed even if the display mode is switched, frequent eye movement of the medical staff is not required.

Also for rows other than the row in which the designated cell is present or columns other than the column in which the designated cell is present, aggregating display to pack the information display cells 51A with the row in which the designated cell is present or the column in which the designated cell is present being located at the center may be performed.

### [Fifth embodiment]

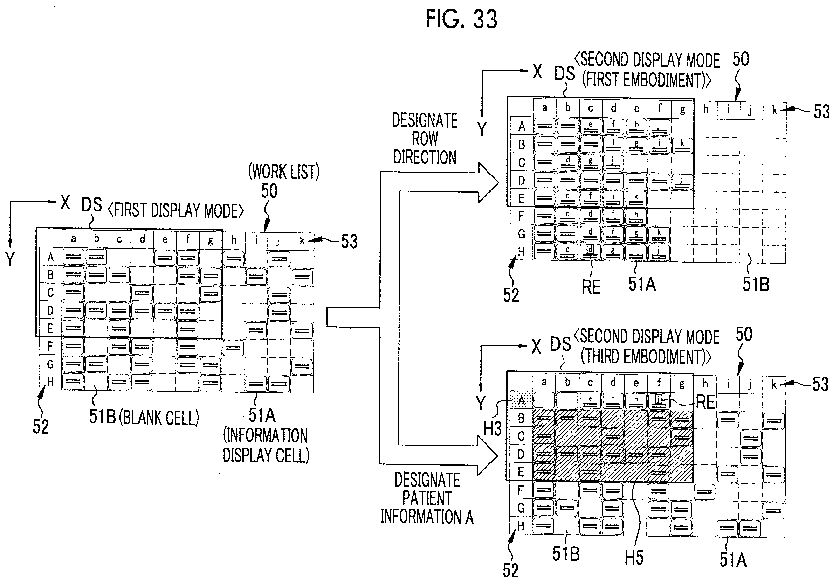

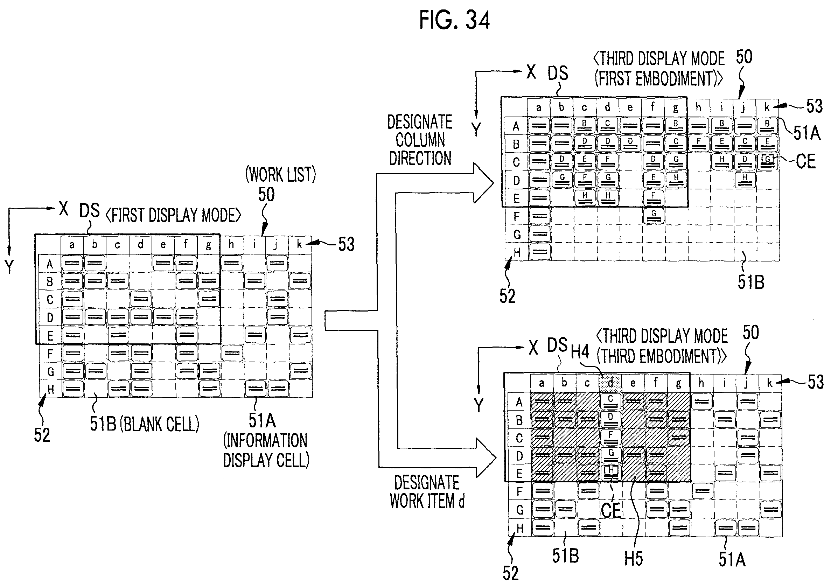

The first to fourth embodiments may be implemented in combination. In a fifth embodiment shown in Figs. 33 and 34, the first and third embodiments are implemented in combination as an example.

In this case, a row direction designation button and a column direction designation button may be provided on the display screen 21, so that it is possible to select each piece of patient information of the patient column 52 and each work item of the item column 53 with the cursor 57.

The display mode switching function includes a function of switching the display mode of the work list 50 to either the second display mode of the first embodiment, in which aggregating display is performed for all of a plurality of rows of the work list 50, or the second display mode of the third embodiment, in which the blank cells 51B in the designated row are deleted, the information display cells 51A in the designated row are aggregatively displayed by being packed together in the row direction, and aggregating display is not performed for rows other than the designated row.

In addition, the display mode switching function includes a function of switching the display mode of the work list 50 to either the third display mode of the first embodiment, in which aggregating display is performed for all of a plurality of columns of the work list 50, or the third display mode of the third embodiment, in which the blank cells 51B in the designated column are deleted, the information display cells 51A in the designated column are aggregatively displayed by being packed together in the column direction, and aggregating display is not performed for columns other than the designated column.

More specifically, as shown in Fig. 33, in a case where the row direction designation button is selected in a case where the display mode of the work list 50 is the first display mode, the display mode of the work list 50 is switched to the second display mode of the first embodiment by the display mode switching function. On the other hand, in a case where patient information is selected in a case where the display mode of the work list 50 is the first display mode, the display mode of the work list 50 is switched to the second display mode of the third embodiment by the display mode switching function.

In addition, as shown in Fig. 34, in a case where the column direction designation button is selected in a case where the display mode of the work list 50 is the first display mode, the display mode of the work list 50 is switched to the third display mode of the first embodiment by the display mode switching function. On the other hand, in a case where a work item is selected in a case where the display mode of the work list 50 is the first display mode, the display mode of the work list 50 is switched to the third display mode of the third embodiment by the display mode switching function.

For example, in the case of checking the work information focusing on taking a look at the general overview of the implementation status of work, such as how much work remains, at the start or end of work of medical facilities, a medical staff member selects the row direction designation button or the column direction designation button to switch the display mode of the work list 50 to the second display mode or the third display mode of the first embodiment.

On the other hand, in the case of checking work information focusing on each patient, for example, at the time of examination of a patient, a medical staff member selects patient information to switch the display mode of the work list 50 to the second display mode of the third embodiment. In addition, in a case where a medical staff member tries to check work information in order to mainly check the work that the medical staff member is in charge of, the medical staff member selects the work item to switch the display mode of the work list 50 to the third display mode of the third embodiment.

Thus, by performing the first and third embodiments in combination, the display mode of the work list 50 can be switched to the second display mode of the first embodiment and the second display mode of the third embodiment or to the third display mode of the first embodiment and the third display mode of the third embodiment. Therefore, the work list 50 of the display mode adapted to various purposes to check the work information can be provided for the viewing of medical staff.

In addition, a case where certain patient information of the patient column 52 is selected may be regarded as the row direction designation and the display mode of the work list 50 may be switched to the second display mode of the first embodiment, or a case where a certain work item of the item column 53 is selected may be regarded as the column direction designation and the display mode of the work list 50 may be switched to the third display mode of the first embodiment. In a case where the information display cell 51 A is selected after direction designation, a row or a column to which the selected information display cell 51A belongs may be determined as a designated row or a designated column, and the display mode of the work list 50 may be switched to the second display mode or the third display mode of the third embodiment.

### [Sixth embodiment]

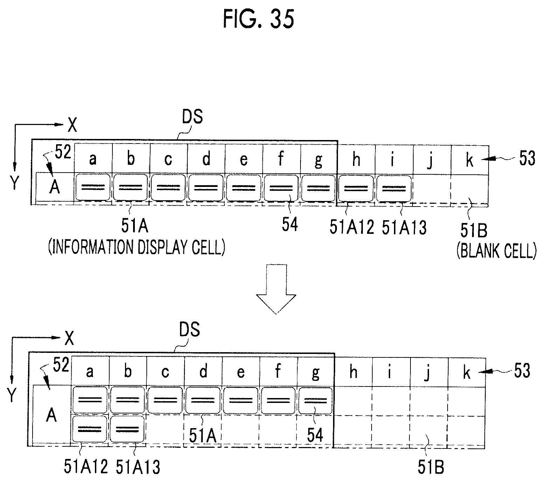

In the second display mode or the third display mode, the information display cells 51 A are aggregatively displayed by being packed together in the row direction or the column direction. However, depending on the number of information display cells 51A, the information display cells 51 A may not fit in the display portion DS at one time in the aggregating display state. In this case, if the scroll bars 56A and 56B are not operated despite the aggregating display of the information display cells 51A, it is not possible to check the work information of all of the information display cells 51 A. Therefore, in a sixth embodiment shown in Fig. 35, in the second display mode or the third display mode, in a case where the aggregatively displayed information display cells 51 A do not fit in the display portion DS at one time, the information display cells 51 A are folded and displayed.

Fig. 35 shows the folded display of the information display cells 51A in the row of the patient information A in the second display mode. As shown in the upper portion, the information display cells 51 A that do not fit in the display portion DS at one time are information display cells 51A12 and 51A13 located at the positions of the work items h and j. In this case, the screen display control unit 43 provides one row of the patient information A in the patient column 52 additionally in the column direction, as shown in the lower portion. Then, the information display cells 51A12 and 51A13 are moved to the additionally provided row, so that the information display cells 51A12 and 51A13 are folded and displayed.

Although not shown, similarly in a case where the aggregatively displayed information display cells 51A do not fit in the display portion DS at one time in the third display mode, a column of the work item is additionally provided so that the information display cells 51 A that do not fit in the display portion DS at one time are folded and displayed.

Since the number of information display cells 51 A that are not displayed in the display portion DS is reduced in this manner, the degree of at-a-glance listing of the work list 50 is further improved. Therefore, it is possible to check a larger amount of work information without operating the scroll bars 56A and 56B.

However, in the case of the first embodiment described above, aggregating display is performed for all rows or all columns. Accordingly, if the information display cells 51A are folded and displayed, the size of the patient column 52 or the item column 53 may be increased in the column direction or the row direction. As a result, there is a concern that the number of information display cells 51 A that do not fit in the display portion DS at one time may be increased on the contrary. For this reason, it is preferable that the present embodiment is implemented in combination with the second and third embodiments in which aggregating display is performed only for the designated row or the designated column.

### [Seventh embodiment]

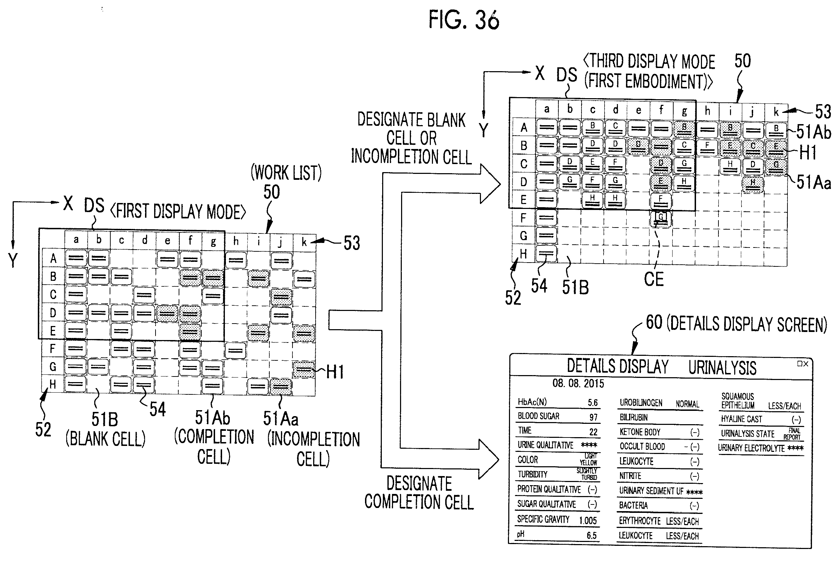

In the fourth embodiment described above, the information display cell 51 A can be selected by the cursor 57, and the information display cells 51 A other than the designated cell are packed together with the designated cell being located at the center. In a seventh embodiment shown in Fig. 36, however, not only the information display cell 51A but also the blank cell 51B can be selected as a designated cell. In a case where the designated cell is the blank cell 51 B or the incompletion cell 51Aa of the information display cells 51A, the information display cells 51 A are packed together in a direction in which the patient array axis is disposed. In a case where the designated cell is the completion cell 51Ab of the information display cells 51A, details of the work are displayed.

In Fig. 36, in a case where the blank cell 51B or the incompletion cell 51Aa is selected in a case where the display mode of the work list 50 is the first display mode, the screen display control unit 43 packs the information display cells 51A in a direction in which the patient array axis is disposed, that is, in the column direction, to switch the display mode of the work list 50 to the third display mode. That is, the selection of the blank cell 51B or the incompletion cell 51 Aa is equivalent to the designation of the column direction.

On the other hand, in a case where the completion cell 51Ab is selected in a case where the display mode of the work list 50 is the first display mode, the screen display control unit 43 generates a details display screen 60 to display the details of the work corresponding to the selected completion cell 51Ab. For example, the details display screen 60 is popup-displayed on the display screen 21. Fig. 36 illustrates the details display screen 60 that displays the examination value of each examination item of urinalysis as the details of the work.

Thus, the information display cells 51A are packed together in a direction in which the patient array axis is disposed in a case where the designated cell is the blank cell 51B or the incompletion cell 51Aa, and the details of the work are displayed in a case where the designated cell is the completion cell 51Ab. Therefore, the work information of each work piece and the number of work pieces can be easily checked, and the details of the work can be easily checked for the work whose progress is "complete".

### [Eighth embodiment]

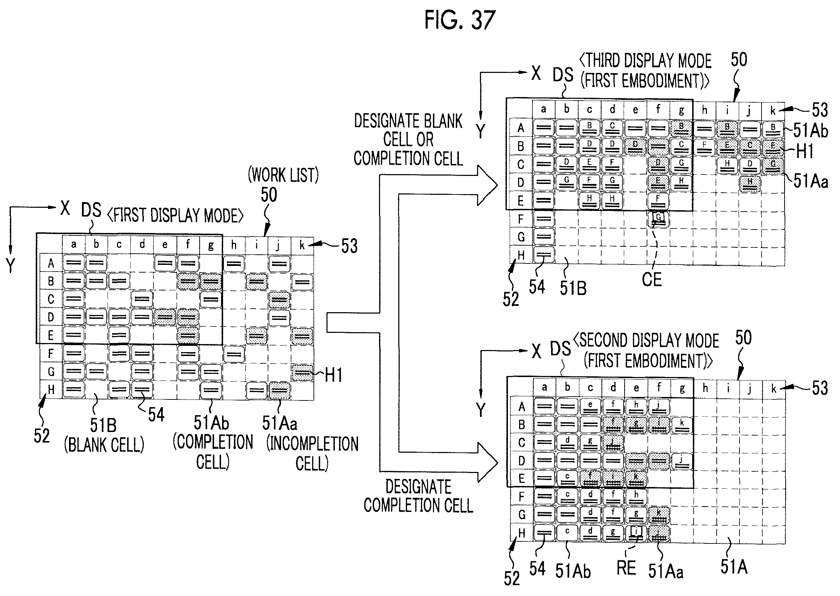

In the seventh embodiment described above, in a case where the designated cell is the completion cell 51 Ab, the details of the work are displayed. In an eighth embodiment shown in Fig. 37, in a case where the designated cell is the completion cell 51Ab, the information display cells 51A are packed together in a direction in which the item array axis is disposed.

In Fig. 37, in a case where the completion cell 51Ab is selected in a case where the display mode of the work list 50 is the first display mode, the screen display control unit 43 packs the information display cells 51A in a direction in which the item array axis is disposed, that is, in the row direction, to switch the display mode of the work list 50 to the second display mode. That is, the selection of the completion cell 51Ab is equivalent to the designation of the row direction.

In a case where the blank cell 51B or the incompletion cell 51Aa is selected in a case where the display mode of the work list 50 is the first display mode, the display mode of the work list 50 is switched to the third display mode as in the seventh embodiment described above.

Thus, the information display cells 51A are packed together in a direction in which the patient array axis is disposed in a case where the designated cell is the blank cell 51 B or the incompletion cell 51 Aa, and the information display cells 51 A are packed together in a direction in which the item array axis is disposed in a case where the designated cell is the completion cell 51Ab. Therefore, it is possible to easily check the work information of each work and each patient or the number of work pieces.

In Figs. 36 and 37, the second display mode and the third display mode of the first embodiment are illustrated. However, a row or a column in which the selected blank cell 51B or the selected incompletion cell 51Aa is present may be determined as a designated row or a designated column to set the second display mode and the third display mode of the second and third embodiments.

The seventh embodiment and the present embodiment may be implemented in combination. In this case, for example, a button for switching between enabling and disabling of the aggregating display function is provided on the display screen 21. Then, the details of the work are displayed in a case where the aggregating display function is disabled and the completion cell 51Ab is selected, and the information display cells 51A are packed together in a direction in which the item array axis is disposed in a case where the aggregating display function is enabled and the completion cell 51Ab is selected.

### [Ninth embodiment]

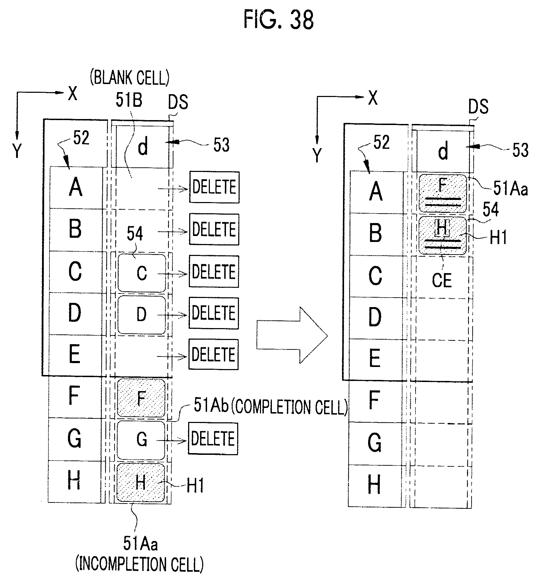

In each of the embodiments described above, the blank cell 51 B is deleted to aggregatively display the information display cells 51 A. In a ninth embodiment shown in Fig. 38, however, not only the blank cell 51B but also the completion cell 51Ab is deleted to aggregatively display only the incompletion cell 51 Aa.

More specifically, as shown on the left side of Fig. 38, the screen display control unit 43 deletes the blank cell 51B and the completion cell 51Ab using the aggregating display function. Then, the incompletion cells 51Aa are shifted to the positions where the blank cell 51B and the completion cell 51Ab are deleted, and finally the incompletion cells 51Aa are collected on the upper side (side of the item column 53) as shown on the right side, thereby realizing the third display mode. In this manner, since only the incompletion cells 51Aa are aggregatively displayed in the work list 50, it is possible to check more easily how much work remains.

In addition, although the case of the third display mode in which the information display cells 51A are packed together in the column direction is illustrated in Fig. 38, the blank cell 51 B and the completion cell 51 Ab may also be deleted similarly for the case of the second display mode.

In addition, only the blank cell 51 B may be deleted in a case where there is designation to pack the information display cells 51A in a direction in which the item array axis is disposed, and the blank cell 51B and the completion cell 51Ab may be deleted in a case where there is designation to pack the information display cell 51A in a direction in which the patient array axis is disposed. In this manner, it is possible to provide the work list 50 of the display mode reflecting the intention of a medical staff member since the medical staff member has an intention to generally check the work information of each patient including the completion cell 51Ab in a case where there is designation to pack the information display cells 51A in a direction in which the item array axis is disposed and the medical staff member has an intention to check how much work remains in a case where there is designation to pack the information display cell 51A in a direction in which the patient array axis is disposed.

### [Tenth embodiment]

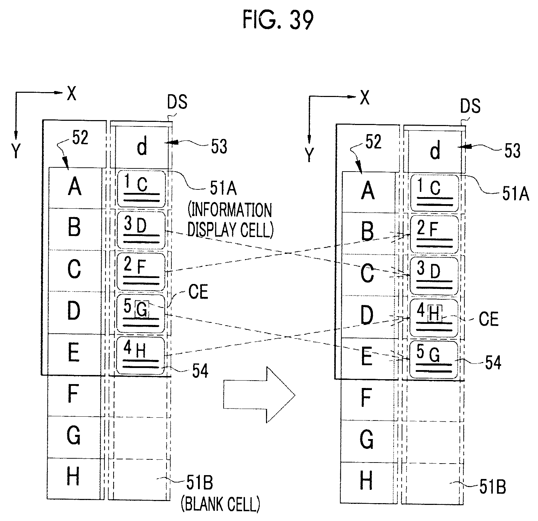

In the patient column 52, pieces of patient information are arranged according to item numbers assigned to the reservation order or the acceptance order of medical examinations, for example. However, the execution order of medical examinations does not necessarily match the item numbers since the execution order of medical examinations may be changed according to the time required for medical examination of each patient, order issuance timing, or the like. For this reason, the information display cells 51 A corresponding to the work items of a medical examination may not be arranged in the execution order. Therefore, in a tenth embodiment shown in Fig. 39, in the case of packing the information display cells 51A in a direction in which the patient array axis is disposed, the information display cells 51A are sorted in the work execution order.

The work execution order is managed, for example, by a hospital information system (HIS) or a radiology information system (RIS) based on the medical data of the order of the electronic medical record 18 of each patient. The work information management unit 45 acquires the data of the work execution order from the HIS or the RIS, and posts the work execution order in each piece of the work information of the work information table 22.

The left side of Fig. 39 shows a case where the information display cells 51A of the work item d are packed together in a direction in which the patient array axis is disposed, that is, in the column direction, to switch the display mode of the work list 50 to the third display mode. In a case where the work execution order of the work item d is 1, 3, 2, 5, and 4 in order from the information display cell 51A on the upper side, the screen display control unit 43 exchanges the position of the information display cell 51 A of the order 3 with the position of the information display cell 51A of the order 2 and exchanges the position of the information display cell 51A of the order 5 with the position of the information display cell 51A of the order 4, so that the display order of the information display cells 51A match the execution order as shown on the right side. In each information display cell 51 A, the patient identification information CE corresponding to the case of the original position is displayed. Numbers showing the work execution order are given for convenience of explanation, but may be actually displayed in the icon 54.

Thus, since the information display cells 51A are sorted in the work execution order, the medical staff can check the work execution order easily. If this is performed during the medical examination of a patient, the execution order of the medical examination whose order has been issued for the patient, who is the target of the medical examination, can be directly transmitted to the patient.

In a case where the present embodiment and the third display mode of the fourth embodiment to pack the information display cells 51 A in the column direction with a designated cell being located at the center are implemented in combination, it is preferable to highlight the designated cell. In this manner, the execution order of the work for the patient corresponding to the designated cell can be seen at a glance.

In addition, the positions of the information display cells 51A may be made to be exchangeable, for example, by drag-and-drop operation. Then, in a case where the positions of the information display cells 51 A are exchanged after sorting, the work execution order may be changed according to this. More specifically, in a case where the positions of the information display cells 51A are exchanged after sorting, a notification of changing the work execution order is transmitted to the HIS or the RIS from the work information management unit 45, and the work execution order may be changed in the HIS or the RIS according to the notification. In this case, ad hoc correspondence is possible. For example, the execution order of the medical examination of an emergency patient can be changed to the earliest order.

In addition, for example, in a case where the blank cell 51B corresponding to the work item of medical examination is selected by the cursor 57, the order of the medical examination may be able to be issued through the display screen 21. For example, a screen for issuing the order of the medical examination may be displayed. In this manner, it is possible to easily issue the order.

In addition, a change of work information, such as a room or medical staff in charge of the work, may be received through the display screen 21. In this case, according to the change of work information, the content of the work information table 22 or the electronic medical record 18 is changed.

In each of the embodiments described above, the item array axis is disposed in the row direction and the patient array axis is displayed in the column direction. However, on the contrary, the patient array axis may be disposed in the row direction, and the item array axis may be disposed in the column direction. In addition, the work information may be directly displayed in the information display cell 51A without providing the icon 54.

The hardware configuration of a computer, which forms the medical assistance server 12 corresponding to the medical assistance device of the invention, can be modified in various ways. For example, in order to improve the processing capacity or reliability, the medical assistance server 12 may be formed by a plurality of server computers that are separated from each other as hardware. For example, the functions of the request receiving unit 41 and the distribution control unit 44, the functions of the screen generation unit 42 and the screen display control unit 43, and the function of the work information management unit 45 may be distributed in three server computers. In this case, the three server computers form the medical assistance device. In addition, the work information DB 12A is provided in a server computer having a function of the work information management unit 45. In addition, the work information DB 12A may be provided separately from the medical assistance device.

In the first embodiment described above, the case has been exemplified in which the medical assistance server 12 generates the display screen 21 and the display screen 21 is reproduced on the client terminal 11 side based on the image data of the display screen 21 from the medical assistance server 12 and is displayed on the display 29A. However, medical data that is the origin of the generation of the display screen 21 may be transmitted to the client terminal 11 from the medical assistance server 12, and the display screen 21 may be generated on the client terminal 11 side. In this case, the screen generation unit 42 is constructed in the CPU 27A of the client terminal 11.

Each functional unit constructed in the CPU 27B of the medical assistance server 12 excluding the distribution control unit 44 may be constructed in the CPU 27A of the client terminal 11, so that the client terminal 11 operates as a medical assistance device. In this case, the request receiving unit 41 receives a distribution instruction and an editing instruction from the GUI control unit 35 instead of the distribution request and the editing request. In addition, the screen generation unit 42 and the screen display control unit 43 output the display screen 21 to the GUI control unit 35.

Thus, the hardware configuration of a computer can be appropriately changed according to the required performance, such as processing capacity, safety, or reliability. Needless to say, in order to ensure the safety or reliability, an application program, such as the medical assistance program 40, may be duplicated or may be stored in a plurality of storage devices in a distributed manner, without being limited to hardware.

Although the embodiment in which the medical assistance server 12 is used in one medical facility has been described in each of the above embodiments, the medical assistance server 12 may be made to be able to be used in a plurality of medical facilities.

In each of the embodiments described above, the medical assistance server 12 is communicably connected to the client terminal 11, which is installed in one medical facility, through the network 13, such as a LAN, and provides the display screen 21 in response to a distribution request from the client terminal 11 or the like. In order to make the medical assistance server 12 available in a plurality of medical facilities, the medical assistance server 12 is communicably connected to each of the client terminals 11 installed in the plurality of medical facilities, for example, through a wide area network (WAN), such as the Internet or a public communication network. Then, the medical assistance server 12 receives distribution requests from the client terminals 11 in the plurality of medical facilities through the WAN, and provides the display screen 21 to the client terminals 11. In the case of using a WAN, it is preferable to construct a virtual private network (VPN) or to use a communication protocol with a high security level, such as hypertext transfer protocol secure (HTTPS), in consideration of information security.

In this case, the electronic medical record 18, the examination image 19, and the medical report 20 are managed for each medical facility. In this case, the installation location and management entity of the medical assistance server 12 may be a data center managed by a company that is different from the medical facilities, or may be one of the plurality of medical facilities, for example.

In the invention, it is also possible to appropriately combine the above-described various embodiments or various modification examples. Without being limited to the embodiments described above, it is needless to say that various configurations can be adopted without departing from the scope of the invention. In addition to the program, the invention also extends to a storage medium that stores the program.

## Claims

1. A medical assistance device, comprising:
a screen generation unit that generates a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed;
a receiving unit that receives designation of either the row direction or the column direction input through the display screen; and
a screen display control unit that has an aggregating display function of aggregatively displaying the information display cells spatially by deleting at least one of the blank cells such that the information display cells are packed together in the row direction or the column direction received by the receiving unit and a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.

2. The medical assistance device according to claim 1,
wherein the receiving unit receives designation for sequentially switching the display mode among the first display mode, the second display mode, and the third display mode by the same input operation through the display screen.

3. The medical assistance device according to claim 1 or 2,
wherein the receiving unit receives at least one of designation of one of a plurality of rows of the work list, designation of one of a plurality of columns of the work list, and designation of one of the plurality of cells.

4. The medical assistance device according to claim 3,
wherein the screen display control unit performs the aggregating display by deleting the blank cells in the one row received by the receiving unit such that the information display cells in the one row are packed together in the row direction and switches the display mode to the second display mode by deleting an entire column regardless of presence or absence of the information display cell for a column in which the blank cell is present, and/or the screen display control unit performs the aggregating display by deleting the blank cells in the one column received by the receiving unit such that the information display cells in the one column are packed together in the column direction and switches the display mode to the third display mode by deleting an entire row regardless of presence or absence of the information display cell for a row in which the blank cell is present.

5. The medical assistance device according to claim 3 or 4,
wherein the screen display control unit performs the aggregating display by deleting the blank cells in the one row received by the receiving unit such that the information display cells in the one row are packed together in the row direction and switches the display mode to the second display mode by not performing the aggregating display for rows other than the one row received by the receiving unit, and/or the screen display control unit performs the aggregating display by deleting the blank cells in the one column received by the receiving unit such that the information display cells in the one column are packed together in the column direction and switches the display mode to the third display mode by not performing the aggregating display for columns other than the one column received by the receiving unit.

6. The medical assistance device according to claim 5,
wherein the screen display control unit highlights the one row aggregatively displayed in the second display mode, and/or highlights the one column aggregatively displayed in the third display mode.

7. The medical assistance device according to any one of claims 3 to 6,
wherein, in a case where the one cell received by the receiving unit is the information display cell, the screen display control unit switches the display mode to the second display mode or the third display mode by packing the information display cells in the row direction or the column direction received by the receiving unit with the one cell being located at a center of the information display cells.

8. The medical assistance device according to any one of claims 1 to 7,
wherein the screen display control unit switches the display mode to the second display mode by performing the aggregating display for all of a plurality of rows of the work list, and/or switches the display mode to the third display mode by performing the aggregating display for all of a plurality of columns of the work list.

9. The medical assistance device according to claim 8 citing claim 5 or 6,
wherein the display mode switching function includes a function of switching the display mode either to the second display mode according to claim 5 or 6 or to the second display mode according to claim 8 and/or a function of switching the display mode either to the third display mode according to claim 5 or 6 or to the third display mode according to claim 8.

10. The medical assistance device according to any one of claims 1 to 9,
wherein, in the second display mode or the third display mode, the screen display control unit displays the work item or the patient identification information corresponding to a case of an original position in the information display cell shifted from the original position in the first display mode due to the aggregating display.

11. The medical assistance device according to any one of claims 1 to 10,
wherein, in the second display mode or the third display mode, in a case where the aggregatively displayed information display cells do not fit in the display screen at one time, the screen display control unit displays the information display cells so as to be folded.

12. The medical assistance device according to any one of claims 1 to 11,
wherein the work information includes a progress of the work,
as the progress, there are "incomplete", which indicates that the work is scheduled to be performed but has not yet been completed, and "complete", which indicates that the work has been completed, and
the information display cells include an incompletion cell in which the progress is "incomplete" and a completion cell in which the progress is "complete".

13. The medical assistance device according to claim 12 citing claim 3,
wherein, in a case where the one cell received by the receiving unit is the blank cell or the incompletion cell, the screen display control unit packs the information display cells in the row direction or the column direction in which the patient array axis is disposed.

14. The medical assistance device according to claim 13,
wherein, in a case where the one cell received by the receiving unit is the completion cell, the screen display control unit displays details of the work.

15. The medical assistance device according to claim 13 or 14,
wherein, in a case where the one cell received by the receiving unit is the completion cell, the screen display control unit packs the information display cells in the row direction or the column direction in which the item array axis is disposed.

16. The medical assistance device according to any one of claims 12 to 15,
wherein the screen display control unit deletes the blank cell and the completion cell, and aggregatively displays only the incompletion cell.

17. The medical assistance device according to any one of claims 1 to 16,
wherein, in a case of packing the information display cells in the row direction or the column direction in which the patient array axis is disposed, the screen display control unit sorts the information display cells in execution order of the work.

18. An operation method of a medical assistance device, comprising:
a screen generation step of generating a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed;
a receiving step of receiving designation of either the row direction or the column direction input through the display screen; and
a screen display control step of executing an aggregating display function of aggregatively displaying the information display cells spatially by deleting at least one of the blank cells such that the information display cells are packed together in the row direction or the column direction received in the receiving step and executing a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.

19. An operation program of a medical assistance device causing a computer to execute:
a screen generation function of generating a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed;
a receiving function of receiving designation of either the row direction or the column direction input through the display screen; and
a screen display control function of executing an aggregating display function of aggregatively displaying the information display cells spatially by deleting at least one of the blank cells such that the information display cells are packed together in the row direction or the column direction received through the receiving function and executing a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.

20. A medical assistance system comprising a medical assistance device and a client terminal connected to the medical assistance device through a network, comprising:
a screen generation unit that generates a display screen of a work list which is configured to include a patient array axis on which patient identification information of a plurality of patients is arranged, an item array axis on which work items that are items of a plurality of work pieces performed on a patient by medical staff are arranged, and a plurality of cells that are intersections between a plurality of pieces of the patient identification information and the plurality of work items, in which one of the patient array axis and the item array axis is disposed in a row direction and the other axis is disposed in a column direction, and in which the cells are information display cells in which work information regarding the work is displayed and blank cells in which the work information is not displayed;
a receiving unit that receives designation of either the row direction or the column direction input through the display screen; and
a screen display control unit that has an aggregating display function of aggregatively displaying the information display cells spatially by deleting at least one of the blank cells such that the information display cells are packed together in the row direction or the column direction received by the receiving unit and a display mode switching function of switching a display mode of the work list to a first display mode in which the blank cells are displayed without being deleted, a second display mode in which the blank cells are deleted such that the information display cells are aggregatively displayed by being packed together in the row direction, and a third display mode in which the blank cells are deleted such that the information display cells are aggregatively displayed by being packed together in the column direction.
